# EUROPEAN PATENT SPECIFICATION

(11) **EP 4 167 962 B1**
(45) Date of publication and mention of the grant of the patent: **02.08.2023**
(21) Application number: 22785698.6
(22) Date of filing: 09.09.2022
(51) Int. Cl.: A61K 9/08, A61K 31/7105, A61P 1/04, A61K 47/02, A61K 47/12, A61K 47/14

(54) **ENEMA FORMULATION**
KLISTIERFORMULIERUNG
FORMULATION DE LAVEMENT

(30) Priority: 09.09.2021 EP 21275126
(43) Date of publication of application: 26.04.2023
(60) Divisional application: 23180657.1
(73) Proprietor: Index Pharmaceuticals AB, 171 65 Solna (SE)
(72) Inventor: CORONA, Franziska Müller, 4402 Frenkendorf (CH); WANNER, Ralf, 79599 Wittlingen (DE); DATHE, Britta, 4106 Therwil (CH); SANDWALL, Pernilla Helena, 167 67 Bromma (SE); JOHANSSON, Christine Dieterich, 186 50 Vallentuna (SE); ALHADEFF, Paul, 147 63 Uttran (SE)
(74) Representative: J A Kemp LLP
(86) International application number: PCT/EP2022/075174
(87) International publication number: WO 2023/036961

(56) References cited:
- WO-A1-2021/037764
- ATREYA RAJA ET AL: "Cobitolimod for moderate-to-severe, left-sided ulcerative colitis (CONDUCT): a phase 2b randomised, double-blind, placebo-controlled, dose-ranging induction trial", THE LANCET - GASTROENTEROLOGY & HEPATOLOGY, ELSEVIER, US, vol. 5, no. 12, 30 November 2020 (2020-11-30), pages 1063-1075, XP009533631, ISSN: 2468-1253, DOI: 10.1016/S2468-1253(20)30301-0 cited in the application
- JANNIN VINCENT ET AL: "Rectal route in the 21st Century to treat children", ADVANCED DRUG DELIVERY REVIEWS, ELSEVIER, AMSTERDAM , NL, vol. 73, 25 May 2014 (2014-05-25), pages 34-49, XP028855089, ISSN: 0169-409X, DOI: 10.1016/J.ADDR.2014.05.012 cited in the application

## Description

### Field of the Invention

The present invention relates to a formulation suitable for rectal administration. The invention further relates to a pre-filled enema comprising said formulation, and to the use of the formulation and enema for treating inflammatory bowel diseases.

### Background of the Invention

Ulcerative colitis (UC) is a disease characterized by chronic inflammation of the rectal and colonic mucosa, affecting the innermost lining in the first stage. The disease is recurrent, with both active and inactive stages that differ in pathology, symptoms and treatment. The underlying cause of UC is not understood, nor is it known what triggers the disease to recur between its inactive and active forms (Kobayashi et al. "Ulcerative colitis." Nat Rev Dis Primers 2020, 6(1): 74; Irvine, E. J. (2008) Inflamm Bowel Dis 14(4): 554-565). Symptoms of active UC include progressive loose stools with blood and increased frequency of bowel movements. Active mucosal inflammation is diagnosed by endoscopy (Harbord M., et al. "Third European evidence-based consensus on diagnosis and management of ulcerative colitis. part 2: current management." J Crohns Colitis 2017, 11(7): 769-7842017).

The stools contain pus, mucous and blood and are often associated with abdominal cramping with urgency to evacuate (tenesmi). Diarrhoea may have an insidious onset or, more rarely, start quite suddenly. In severe cases the symptoms may include fever and general malaise. In severe stages, deep inflammation of the bowel wall may develop with abdominal tenderness, tachycardia, fever and risk of bowel perforation. Furthermore, patients with UC may suffer extra intestinal manifestations such as arthralgia and arthritis, erythema nodosum, pyoderma gangrenosum and inflammation in the eyes. In the case of remission or inactive UC, patients are usually free of bowel symptoms.

The extent of inflamed and damaged mucosa differs among patients with UC. UC that affects only the rectum is termed ulcerative proctitis. The condition is referred to as distal or left-sided colitis when inflammatory changes are present in the left side of the colon up to the splenic flexure. In extensive UC the transverse colon is also affected, and pancolitis designates a disease involving the entire colon.

Active mucosal inflammation is diagnosed by endoscopy and is characterized by a loss of vascular patterning, oedema, petechia, spontaneous bleeding and fibrinous exudates. The endoscopic picture is that of continuous inflammation, starting in the rectum and extending proximally to a variable extent into the colon. Biopsies obtained at endoscopy and subjected to histological examination help to diagnose the condition. Infectious causes, including clostridium difficile, camphylobacter, Salmonella and Shigella, may mimic UC and can be excluded by stool cultures.

The medical management of UC is divided into treatment of active disease and maintenance of remission.

The medical management of UC is divided into treatment of active disease and maintenance of remission. The treatment of patients with active UC aims to reduce inflammation and promote colon healing and mucosal recovery. In most cases the disease may be controlled with conventional drugs including SP, 5-ASA ((Sutherland et al. (1987) Gastroenterology 92: 1894-1898) and GCS (Domenech et al (2014). Dig Dis 32(4): 320-327)). Left-sided disease, limited to the rectum and the recto-sigmoid area, is often treated with rectal formulations of 5-ASA and GCS. When the extent is more proximal and/or the symptoms more severe, oral GCS is also needed. GCS are generally used to treat disease flare-ups and are not recommended for maintenance of remission since there are significant side effects (such as immunodeficiency, adrenal insufficiency, hyperglycemia, hypothalamic pituitary adrenal axis suppression, steroid-induced osteoporosis: reduced bone density, weight gain, muscle break down,) inherent in long-term use and the possible development of steroid dependent disease. Glucocorticoid drugs act non selectively, so in the long run they may impair many healthy anabolic processes. As a result, maintenance treatment with systemic GCS is not advised (Prantera, C. and S. Marconi (2013) Therap Adv Gastroenterol 6(2): 137-156). New formulations of GCS have been developed with the aim of limiting systemic activity and reducing GCS AEs. For patients who become refractory to GCS and suffering from severe or moderately severe flares of UC, immunomodulatory agents such as azathioprine/6-mercaptopurine and cyclosporine are sometimes used. However, immunomodulators are slow-acting and the induction of remission in these patients is often temporary (Ford et al. (2011) Am J Gastroenterol 106(4): 630-642).

Treatment options for UC have rapidly expanded in recent years and now include multiple biologic agents and the small molecule drugs tofacitinib and Zeposia/ozanimod in addition to prior medication options (see Ford et al. (2011) Am J Gastroenterol 106(4): 630-642; Fausel, R. and A. Afzali (2015) Ther Clin Risk Manag 11: 63-73; Roda et al. (2016) Clin Transl Gastroenterol 7: e135; Fiorino et al. (2017) Expert Opin Drug Saf 16(4): 437-443;Troncone and Monteleone (2017) Expert Opin Drug Saf 16(7): 779-789; Duijvestein et al. (2018) Curr Treat Options Gastroenterol 16(1): 129-146; Verstockt et al. (2018) J Gastroenterol 53(5): 585-590; Troncone et al. (2020) Clin Exp Gastroenterol 13: 131-139; and also recent results for Zeposia/ozanimod at https://www.healthline.com/health-news/fda-approves-new-drug-for-ulcerative-colitis#Clinical-trial-results).

Three TNFα inhibitors currently approved for the treatment of moderate to severe UC are infliximab, adalimumab, and golimumab. These agents bind TNFα, neutralize its activity, and prevent it from binding to its receptor. Infliximab and adalimumab have also been shown to induce apoptosis of activated T cells and macrophages. All three TNFα inhibitors carry potential risks associated with their use, and should be avoided in patients with uncontrolled infections, advanced heart failure, neurologic conditions and in patients with a history of malignancy, due to the potential risk of accelerating the tumour growth. Serious infections occur in 2%-4% of patients treated with anti TNFα antibodies (Fausel, R. and A. Afzali (2015) Ther Clin Risk Manag 11: 63-73). Other potential adverse effects of anti-TNFα therapy include acute and serious infusion reactions, including anaphylaxis, convulsions, and hypotension. Injection site reactions and rare anaphylactic reactions can also occur with subcutaneously administered anti-TNFα agents. Other possible adverse effects include neutropenia, hepatotoxicity, serum sickness, leukocytoclastic vasculitis, rash including psoriasiform rash, and induction of autoimmunity. Approximately 50% of patients receiving infliximab develop antibodies after 2 years (Fausel, R. and A. Afzali (2015) Ther Clin Risk Manag 11: 63-73).

All three anti-TNα agents have been shown to be effective in inducing and maintaining clinical response and remission in patients with UC, with fairly comparable safety profiles. Regardless of the anti-TNFα agent chosen, combination therapy with azathioprine is likely more effective for inducing remission than anti-TNFα monotherapy. However, there are still up to 50% of patients receiving anti-TNFα agents who fail to respond to induction dosing, and even more patients who lose response to the anti-TNFα agent over time (Fausel, R. and A. Afzali (2015) Ther Clin Risk Manag 11: 63-73). Vedolizumab, an α4β7 integrin inhibitor was, in 2014, approved for the treatment of UC. In the GEMINI 1 trial, vedolizumab was found to be more effective than placebo for inducing and maintaining clinical response, clinical remission, and mucosal healing (Feagan et al. (2013) N Engl J Med 369(8): 699-710.). In theory, because of the unique location of the α4β7 integrin on lymphocytes homing to the gut, it may be expected that vedolizumab will have a reduced risk of systemic infections and malignancy compared with systemically acting agents. In the GEMINI 1 trial, no important differences were observed between the study groups in terms of AEs.

Tofacitinib is an oral, small-molecule, ATP-competitive reversible inhibitor of the JAK-1 and JAK-3, and, at high concentrations, TYK2 and JAK-2 pathways as well. Interestingly, tofacitinib is rapidly absorbed after oral intake with a time to peak concentration of 30 min, thus allowing a more comfortable route of administration for the patient and a quicker onset of effect than the majority of other drugs used in IBD (D'Amico et al. (2019) Therap Adv Gastroenterol 12: 1756284819848631). The tofacitinib dosing regimen for adults with moderate to severe UC is 10 mg BID for at least eight weeks, followed by 5 mg BID or 10 mg BID. This dosing regimen is based on data from three pivotal phase III studies from the OCTAVE clinical development programme. All three studies met their respective primary endpoints, with a significantly greater proportion of patients in remission at week 8 in the induction studies and at week 52 in the maintenance study with tofacitinib compared to placebo (Paschos et al. (2018) Ann Gastroenterol 31(5): 572-582).

However, adverse events observed in the tofacitinib UC clinical programme included serious infection, including herpes zoster, opportunistic infections, malignancies (including non-melanoma skin cancer and lymphoproliferative disorders), gastrointestinal perforation and laboratory abnormalities. EMA and FDA concluded that tofacitinib could increase the risk of blood clots in the lungs and in deep veins in patients, who are already at high risk, and recommended that tofacitinib should be used with caution in all patients at high risk of blood clots (FDA (2019). Safety trial finds risk of blood clots in the lungs and death with higher dose of tofacitinib (Xeljanz, Xeljanz XR) in rheumatoid arthritis patients; FDA to investigate, U.S. Food and Drug Administration; EMA confirms Xeljanz to be used with caution in patients at high risk of blood clots, European Medicines Agency. EMA/92517/2020).

Zeposia or ozanimod is a sphingosine 1-phosphate (S1P) receptor modulator belonging to the "small molecule" class of medicines. Ozanimod binds tightly and specifically to S1P receptors 1 and 5. By binding to these receptors, ozanimod is thought to block the ability of lymphocytes to escape from lymph nodes, which traps them in the lymph nodes and reduces their numbers in the blood, central nervous system, and in inflamed tissues. The FDA recently approved ozanimod for treatment of moderate to severe active ulcerative colitis in adults. Because ozanimod modulates the function of lymphocytes it can increase the risk of infection. The biggest adverse effects are in certain patients who had myocardial infarction in the last 6 months, or other cardiac issues like unstable angina or heart failure. Use of ozanimod with antineoplastic, non-corticosteroid immunosuppressive, or immune-modulating therapies is also expected to increase the risk of immunosuppression, and these treatments may be contraindicated. The use of live attenuated vaccines should also be avoided during and for 3 months after treatment with ozanimod.

Ustekinumab, a fully human IgG1κ monoclonal antibody that binds with specificity to the shared p40 protein subunit of human cytokines IL12 and IL23, is the most recently approved treatment for moderate to severe active UC in adults.

In the phase III UNIFI programme, patients with moderately to severely active UC received an iv induction dose of ustekinumab (130 mg or a weight-range-based dose approximating 6 mg/kg) or placebo, and those with clinical response at week 8 were re-randomized to receive sc maintenance injections of 90 mg ustekinumab (every 8 or 12 weeks) or placebo. The percentage of patients in clinical remission at week 8, and at week 44, was significantly higher among those who received treatment with ustekinumab than with placebo (Sands et al. (2019) N Engl J Med 381(13): 1201-1214).

The most common side effects of ustekinumab include upper respiratory tract infection, nasopharyngitis, dizziness, headache, oropharyngeal pain, diarrhoea, nausea, vomiting, pruritus, back pain, myalgia, arthralgia, fatigue, injection site erythema and injection site pain.

While TNF inhibitors and vedolizumab are widely used and positioned as third line therapies in UC, it remains to be seen how the novel JAK and IL12 and IL23 directed therapies will be positioned in the treatment regimen for UC.

Despite the advanced therapies described above, UC patients with chronically active disease still are at risk of becoming treatment-refractory which poses a serious medical challenge and the only remaining course of action is colectomy. The 5- and 10-year cumulative risk of colectomy is 10 to 15% in left sided colitis (Fumery et al. (2018) Clin Gastroenterol Hepatol., 16(3): 343-356). A total colectomy is a potentially curative option in severe UC but is a life-changing operation that entails risk of complications, such as pouch failure, pelvic sepsis, infertility in women and nocturnal faecal soiling. Therefore, surgery is usually reserved for patients with severe refractory disease, surgical emergencies, or patients with colorectal dysplasia or cancer (Hoivik et al. (2012) Inflamm Bowel Dis 18(8): 1540-1549).

An emergent third line treatment for UC is cobitolimod (Kappaproct/DIMS0150), a modified single strand deoxyribonucleic acid (DNA)-based synthetic oligonucleotide of 19 bases in length. Cobitolimod has the sequence 5'- G*G*A*ACAGTTCGTCCAT*G*G*C-3' (SEQ ID NO:1), wherein the CG dinucleotide is unmethylated. The star represents a phosphorothioated linkage.

Cobitolimod functions as an immunomodulatory agent by targeting the Toll-like receptor 9 (TLR9) present in immune cells or on the surface of epithelial cells. These immune cells (i.e., B-cells and plasmacytoid dendritic cell (pDCs) and epithelial cells) reside in high abundance in colonic and nasal mucosa of the lumen. The immune system is the key mediator of the changes of UC. The mucosa of the colon and rectum of patients with UC is chronically inflamed and contains active immune cells. Cobitolimod may be topically administered in the region of inflammation, which places the drug in close contact with a high number of intended target cells, ensuring that the drug will reach an area rich in TLR9 expressing cells. The activation of these cells by cobitolimod induces various cytokines, such as type I interferons and interleukin 10 (IL-10) which are classical anti- inflammatory cytokines and are believed to be important factors for the clinical effect of cobitolimod.

The clinical efficacy of cobitolimod has been demonstrated in the "COLLECT" (CSUC-01/10 ) clinical trial, which involved the administration to patients of 30 mg doses of cobitolimod, at 4 week intervals and also in the "CONDUCT" (CSUC-01/16 ) clinical trial, which involved testing different dosage regimes. The details of the "COLLECT" trial were published in Journal of Crohns and Colitis (Atreya et al. J Crohns Colitis, 2016 May 20) and the details of the "CONDUCT" clinical trial were published in The Lancet Gastroenterology and Hepatology (Atreya et al 2020. Lancet Gastroenterol Hepatol. 2020 Dec;5(12):1063-1075). Overall, data on cobitolimod supports a positive benefit-risk assessment for patients with chronic UC. Cobitolimod is safe and well tolerated and has been shown to be effective to induce clinical response and remission in patients with chronic UC, as well as symptomatic and endoscopic remission in patients with treatment refractory, moderate to severe chronic UC.

In the COLLECT study, which involved administration of a relatively low (30mg) dose of cobitolimod, topical administration of cobitolimod was performed using a spray catheter device, administered during an endoscopy. This is an invasive medical procedure which is necessarily carried out by a medical professional. Further, before the topical administration of the cobitolimod to the patients, the colon of each patient was cleaned to remove faecal matter. That was done to enable the cobitolimod to reach the intestinal epithelial cells within the colon and to enable the endoscopist to view the colonic mucosa. Various methods of cleaning the colon are known in the art. For instance, prior to endoscopy, the patient may need to drink up to 4L of fluid starting the day before the endoscopy. In addition to an active cleansing agent, bowel preparation may consist of the patient eating a restricted diet. Such a diet may involve avoiding seeds and corn, and may be recommended for several days prior to the endoscopy. Typically, only clear fluids are permitted on the day preceding the procedure. There are also drugs available for cleaning the colon, e.g. Plenvu, which is approximately 1 L liquid drug which is designed to replace the traditional, poor-tasting bowel cleaning liquid. In summary, depending on the laxative agent selected, the patient typically needs to drink around 1-4 L of fluid prior to endoscopy. This is an uncomfortable procedure and is disruptive and inconvenient for the patient.

The cobitolimod itself was supplied as a solution of cobitolimod in water in aseptically filled glass vials. Administration to the patient was done by medical care personnel as it required some preparation such as dilution of the solution and transfer to an appropriate syringe for administration via the spray catheter.

As noted above, patients suffering from chronic ulcerative colitis, who are in an active disease state and refractory to known treatments pose a serious medical challenge and often the only remaining course of action is colectomy. For this reason, patients will tolerate medical intervention which requires both colonic cleaning to remove faecal matter and topical administration via spray catheter, despite the inconvenience and discomfort involved in such invasive procedures.

In the CONDUCT study cobitolimod was supplied in single-use glass vials, containing 50 mL solution. Prior to dosing, the study personnel transferred the solution to a plastic enema and then administered the cobitolimod solution to the patient who was lying in a left-sided position. This procedure did not require a colonoscopy, however it was still administered by the site personnel and the enema was not optimal for either storage or self-administration.

However, it would be therapeutically desirable to provide a topical treatment for ulcerative colitis patients which, preferably, can be self-administered by the patient and preferably does not require any cleaning steps prior to administration. In this regard, it would be desirable to provide cobitolimod in a format which is suitable for self-administration and which is stable to distribution and storage conditions, thereby permitting the administration of the drug in the comfort of the patient's house, without the need for professional medical intervention. Such a treatment would also be beneficial for patients who are taking cobitolimod as a maintenance therapy (i.e. to keep them in remission), who could be taking cobitolimod for extended periods of time e.g. several months or even years. Further, providing cobitolimod in a form suitable for self-administration at home would be desirable because this would avoid a patient needing to take multiple trips to medical facilities and taking up the time of medical professionals on non-urgent work, thereby helping to minimise strain on health care systems, in particular during public health crises such as the COVID-19 pandemic.

WO2021/037764 describes the use of an oligonucleotide comprising the sequence 5'-GGAACAGTTCGTCCATGGC-3' (SEQ O ID NO:2) for use in the treatment of an inflammatory bowel disease in a human subject via topical administration to the colon, wherein the subject has not been subjected to colonic cleaning prior to said administration. Atreya et al "Cobitolimod for moderate-to-severe, left-sided ulcerative colitis (CONDUCT): a phase 2b randomised, double-blind, placebo controlled dose ranging induction trial" The Lancet - Gastroenterology & Hepatology, vol. 5, no. 12, 2020 pages 1063-1075 provides further details of the CONDUCT clinical trial mentioned above. Jannin et al "Rectal route in the 21st Century to treat children" Advanced Drug Delivery Reviews, vol. 73, 2014, pages 34-49 is a review of rectally administered medicines used to treat children.

### Summary of the Invention

Studies performed by the inventors have identified that cobitolimod is susceptible to certain degradation mechanisms, for instance oxidation of the phosphorothioate linkages, in response to various stimuli (light, elevated temperature, oxidation, acids) or microbial growth. As mentioned above, the earlier clinical studies have used aseptically-filled glass vials to store the cobitolimod preparations prior to administration. Glass vials are impermeable and unreactive, therefore preventing degradation of the cobitolimod. However, the glass vials used in previous studies are not suitable for self-administration by the patient. As explained above, when cobitolimod is supplied in this format, further steps must be taken before the drug is administered, such as dilution of the solution and/or transfer to an appropriate container for administration. Typically, such administration requires professional medical assistance and more complex medical equipment (e.g. colonoscopes) thereby requiring the patient to travel to a hospital or clinic.

Pre-filled enemas, which the patient can administer themselves would be a preferred option. However, the containers used for such enemas are typically (non-sterile) plastic, which creates further problems because plastic containers are far more permeable to the surrounding gases than glass. During long-term storage, the permeation of gases such as oxygen and carbon dioxide into the container causes degradation of the drug and reduced efficacy.

It has now surprisingly been found that a particular formulation of cobitolimod provides compositions which are stable to long term storage. In particular, the formulations of the invention are resistant to drug degradation and microbial growth. Further the formulation of the invention is stable when used in plastic containers, which are more permeable to the gases in the surrounding air (e.g. oxygen), which may degrade the oligonucleotide, unlike in glass vials. The formulations in question include a buffer system as well as preservatives. The buffer system used was found to be compatible with cobitolimod whilst also being suitable for rectal administration. In particular, the buffer system is able to keep cobitolimod close to physiological pH for extended periods of time, e.g. up to one year, and only requires low preservative concentrations to suppress microbial growth.

The invention also relates to a pre-filled enema with the formulation of the invention, which provides a product which is stable during long term storage. The enema container is well-adapted to administer the formulation to the patient with minimal discomfort and ensuring that substantially all of the formulation is expelled.

The present invention therefore provides an aqueous formulation suitable for rectal administration comprising (i) an oligonucleotide comprising the sequence 5'-GGAACAGTTCGTCCATGGC-3' (SEQ ID NO:2), (ii) at least one preservative and (iii) a buffer comprising citric acid and a phosphate salt.

The invention also provides a pre-filled enema suitable for self-administration, said pre-filled enema comprising:
(a) a container; and within the container
(b) the formulation as described herein.

The invention also provides a pack comprising one or more pre-filled enemas as described herein.

The invention also provides a formulation as described herein for use in the treatment of an inflammatory bowel disease, preferably wherein said inflammatory bowel disease is ulcerative colitis or Crohn's disease.

The invention also provides a formulation as described herein for use in a method of treating an inflammatory bowel disease in a subject, the method comprising rectally administering to said subject the formulation, preferably wherein said inflammatory bowel disease is ulcerative colitis or Crohn's disease.

The invention also provides the use of a formulation as described herein for the manufacture of a medicament for treatment of inflammatory bowel disease, preferably wherein the inflammatory bowel disease is ulcerative colitis or Crohn's disease.

### Detailed Description of the Invention

### Formulation

The invention provides an aqueous formulation suitable for rectal administration comprising (i) an oligonucleotide comprising the sequence 5'-GGAACAGTTCGTCCATGGC-3' (SEQ ID NO:2), (ii) at least one preservative and (iii) a buffer comprising citric acid and a phosphate salt.

As used herein, the term "aqueous formulation" refers to a formulation in which the various ingredients (oligonucleotide, preservative and the chemicals used to provide the buffer) are dissolved in an aqueous solvent. An aqueous solvent comprises water, typically in an amount of at least 10% by weight of the aqueous solvent, preferably in an amount of at least 25% by weight of the aqueous solvent, more preferably in an amount of at least 50% by weight of the aqueous solvent, more preferably in an amount of at least 75% by weight of the aqueous solvent. For instance, the aqueous solvent may be water i.e. the aqueous solvent may consist of water.

As used herein, the phrase "suitable for rectal administration" means that the formulation or pre-filled enema may be safely administered rectally i.e. without causing damage to the rectum. More detail on the various considerations for rectal formulations may be found in Hua "Physiological and Pharmaceutical Considerations for Rectal Drug Formulations" Frontiers in Pharmacology (2019), vol 10, 1196 and in Jannin et al, "Rectal route in the 21st Century to treat children" Advanced Drug Delivery Review (2014), 73, 34-49.

As used herein, the term "oligonucleotide" refers to a polynucleoside formed from a plurality of linked individual nucleoside units. Such oligonucleotides can be obtained from existing nucleic acid sources, including genomic DNA or cDNA, plasmids, vectors, or bacterial DNA, but are preferably produced by synthetic methods. The nucleoside residues can be coupled to each other by any of the numerous known internucleoside linkages. Such internucleoside linkages include, without limitation, the natural internucleoside phosphodiester bond or indeed modified internucleosides such as, but not limited to, phosphorothioate, phosphorodithioate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide" also encompasses polynucleosides having one or more stereospecific internucleoside linkages (e. g., (Rp)- or (Sp)-phosphorothioate, alkylphosphonate, or phosphotriester linkages). As used herein, the terms "oligonucleotide" and "dinucleotide" are expressly intended to include polynucleosides and dinucleosides having any such internucleoside linkage, whether or not the linkage comprises a phosphate group. In certain preferred embodiments, these internucleoside linkages may be phosphodiester, phosphorothioate, or phosphorodithioate linkages, or combinations thereof.

The term "oligonucleotide" also encompasses polynucleosides having additional substituents including, without limitation, protein groups, lipophilic groups, intercalating agents, diamines, folic acid, cholesterol and adamantane. The term "oligonucleotide" also encompasses any other nucleobase containing polymer, including, without limitation, peptide nucleic acids (PNA), peptide nucleic acids with phosphate groups (PHONA), locked nucleic acids (LNA), morpholino-backbone oligonucleotides, and oligonucleotides having backbone sections with alkyl linkers or amino linkers. The alkyl linker may be branched or unbranched, substituted or unsubstituted, and chirally pure or a racemic mixture.

The oligonucleotides may include naturally occurring nucleosides, modified nucleosides, or mixtures thereof. As used herein, the term "modified nucleoside" is a nucleoside that includes a modified heterocyclic base, a modified sugar moiety, or a combination thereof. In some embodiments, the modified nucleoside is a non-natural pyrimidine or purine nucleoside, as herein described. In some embodiments, the modified nucleoside is a 2'-substituted ribonucleoside, an arabinonucleoside or a 2'-deoxy-2'-substituted-arabinoside.

As used herein, the term "a hybrid oligonucleotide" is an oligonucleotide having more than one type of nucleoside.

Herein, the term "oligonucleotide" includes hybrid and chimeric oligonucleotides. A "chimeric oligonucleotide" is an oligonucleotide having more than one type of internucleoside linkage within its sequence structure. One preferred example of such a chimeric oligonucleotide is a chimeric oligonucleotide comprising a phosphorothioate, phosphodiester or phosphorodithioate region and nonionic linkages such as alkylphosphonate or alkylphosphonothioate linkages (US5635377 and US5366878).

Herein, the term "oligonucleotide" also includes circularized variants and circular oligonucleotides.

Preferably, the oligonucleotide comprises at least one naturally occurring phosphodiester, or one modified phosphorothioate, or phosphorodithioate internucleoside linkage, however preferred linkages or indeed backbone modifications including, without limitation, methylphosphonates, methylphosphonothioates, phosphotriesters, phosphothiotriesters, phosphorothioates, phosphorodithioates, triester prodrugs, sulfones, sulfonamides, sulfamates, formacetal, N-methylhydroxylamine, 2' OMe (OxyMethyl group at 2'position), carbonate, carbamate, morpholino, boranophosphonate, phosphoramidates, especially primary amino-phosphoramidates, N3 phosphoramidates and N5 phosphoramidates, and stereospecific linkages (e. g., (Rp)-or (Sp)-phosphorothioate, alkylphosphonate, or phosphotriester linkages) are also envisaged.

The sugar moiety of the nucleoside can be a non-naturally occurring sugar moiety. Herein, a "naturally occurring sugar moiety" is a sugar moiety that occurs naturally as part of a nucleic acid, e. g., ribose and 2'- deoxyribose, and a "non-naturally occurring sugar moiety" is any sugar that does not occur naturally as part of a nucleic acid, but which can be used in the backbone for an oligonucleotide, for example but not limited to hexose. Arabinose and arabinose derivatives are examples of preferred sugar moieties.

Modified or substituted oligonucleotides are often preferred over native forms because of desirable properties such as, for example, enhanced cellular uptake, enhanced affinity for nucleic acid target and increased stability in the presence of nucleases. An oligonucleotide is usually comprised of more than ten (10) and up to one hundred (100) or more deoxyribonucleotides or ribonucelotides, although preferably between about eight (8) and about forty (40), most preferably between about eight (8) and about twenty (20). The exact size will depend on many factors, which in turn depends on the ultimate function or use of the oligonucleotide. The oligonucleotide may be generated in any manner, including chemical synthesis, DNA replication, reverse transcription, or a combination thereof.

The oligonucleotide in the formulation of the invention comprises the sequence 5'-GGAACAGTTCGTCCATGGC-3' (SEQ ID NO:2). The oligonucleotide in the formulation of the invention may consist of the sequence 5'-GGAACAGTTCGTCCATGGC-3' (SEQ ID NO:2), i.e. the oligonucleotide may be an oligonucleotide having the sequence 5'-GGAACAGTTCGTCCATGGC-3' (SEQ ID NO:2).

Typically, at least one CG dinucleotide in the oligonucleotide is unmethylated. The oligonucleotide may therefore have the sequence 5'-GGAACAGTTCGTCCATGGC-3' (SEQ ID NO:2), wherein the CG dinucleotide is unmethylated.

Typically, at least one nucleotide in said oligonucleotide has a phosphate backbone modification. The backbone modification is typically a phosphorothioate or a phosphorodithioate modification. Typically, the backbone modification is located in the 5'- and/or the 3'- end of said oligonucleotide.

Phosphorothioate linkages can be illustrated with asterisks (*) in a sequence, e.g. in the sequence: 5'-G*G*A*ACAGTTCGTCCAT*G*G*C-3' (SEQ ID NO:1), wherein the CG dinucleotide is unmethylated. Preferably, the oligonucleotide has the sequence 5'-G*G*A*ACAGTTCGTCCAT*G*G*C-3' (SEQ ID NO:1), wherein the CG dinucleotide is unmethylated i.e. the oligonucleotide in the formulation of the invention may consist of the sequence 5'-G*G*A*ACAGTTCGTCCAT*G*G*C-3' (SEQ ID NO:1), wherein the CG dinucleotide is unmethylated.

Thus, typically the oligonucleotide is cobitolimod.

The concentration of the oligonucleotide (preferably cobitolimod) used is not particularly limited and may be any concentration that delivers a therapeutic benefit to the patient. Typically, the oligonucleotide (preferably cobitolimod) is present in the formulation in a concentration of at least 0.1 mg/mL, for instance at least 0.5 mg/mL, or at least 1 mg/mL, at least 1.5 mg/mL, at least 2.0 mg/mL or at least 2.5 mg/mL. The oligonucleotide (preferably cobitolimod) may be present in the formulation in an amount of less than 25 mg/mL, typically less than 20 mg/mL, for instance less than 15 mg/mL. Thus, typically, the oligonucleotide (preferably cobitolimod) is present in the formulation in an amount from 0.1 to 25 mg/mL, preferably from 0.5 to 20 mg/mL. Typically, the oligonucleotide (preferably cobitolimod) is present in the formulation at a concentration of from 0.5 to 15 mg/mL. Preferably the oligonucleotide is present in the formulation at a concentration of from 2.5 to 12.5 mg/mL.

The oligonucleotide (preferably cobitolimod) may be present in the formulation at a concentration of about 2.5 mg/mL. The oligonucleotide (preferably cobitolimod) may be present in the formulation at a concentration of about 5 mg/mL. The oligonucleotide (preferably cobitolimod) may be present in the formulation at a concentration of about 10 mg/mL.

The at least one preservative may be any preservative known to the skilled person, as long as the preservative can suppress microbial growth without degrading the oligonucleotide. The formulation may comprise one type of preservative. Alternatively, the formulation may comprise two or three types of preservatives.

Typically, the total concentration of preservatives in the formulation is less than 1% by weight of the formulation, typically less than 0.5% by weight of the formulation, preferably less than 0.2% by weight of the formulation. The total concentration of preservatives in the formulation may be from 0.001 to 1% by weight of the formulation, for instance from 0.005 to 0.5% by weight of the formulation, preferably from 0.01 to 0.2% by weight of the formulation.

Typically, the at least one preservative comprises one or more parabens. The at least one preservative may consist of one or more parabens. The term "parabens" is well known in the field of formulation chemistry, and typically refers to parahydroxybenzoates or esters of parahydroxybenzoic acid.

Suitable parabens would be known to the skilled person. For instance, the preservative may comprise one or more parabens selected from methylparaben, ethylparaben, propylparaben, butylparaben, heptylparaben, isobutylparaben, isopropylparaben, benzylparaben and salts thereof. The preservative may comprise one or more parabens selected from methylparaben, ethylparaben, propylparaben, butylparaben heptylparaben, isobutylparaben, isopropylparaben, benzylparaben and sodium salts thereof.

Typically, the at least one preservative comprises Na-methylparaben and/or Na-propylparaben. Preferably, the at least one preservative is selected from the group consisting of Na-methylparaben, Na-propylparaben and mixtures thereof. For instance, the at least one preservative may consist of a mixture of Na-methylparaben and Na-propylparaben.

The total concentration of the parabens is typically less than 1% by weight of the formulation, typically less than 0.5% by weight of the formulation, preferably less than 0.2% by weight of the formulation. The total concentration of parabens in the formulation may be from 0.001 to 1% by weight of the formulation, for instance from 0.005 to 0.5% by weight of the formulation, preferably from 0.01 to 0.2% by weight of the formulation. Preferably, the formulation comprises from 0.01 to 0.2% by weight of a mixture of Na-methylparaben and Na-propylparaben.

The buffer comprises citric acid and a phosphate salt. The skilled person would be aware of how to prepare a buffer at a certain pH by mixing stock solutions of the two components. Preferably, the phosphate salt is disodium hydrogenphosphate (NazHPOa).

The formulation typically has a pH of at least 6.0, preferably of at least 6.5. The formulation typically has a pH of no more than 8.5, preferably of no more than 8. Typically, the formulation has a pH in the range of 6.0 to 8.0 i.e. the buffer maintains the pH of the formulation in the range of 6.0 to 8.0. Preferably the formulation has a pH in the range of 6.5 to 8.0 i.e. the buffer maintains the pH of the formulation in the range of 6.5 to 8.0.

Thus, typically the formulation comprises (i) an oligonucleotide which is cobitolimod, (ii) at least one preservative comprising one or more parabens and (iii) a buffer comprising citric acid and a phosphate salt. For instance, the formulation may comprise (i) an oligonucleotide which is cobitolimod, (ii) at least one preservative comprising one or more parabens and (iii) a buffer comprising citric acid and disodium hydrogenphosphate (Na₂HPO₄).

The formulation may comprise (i) an oligonucleotide which is cobitolimod in an amount from 0.1 to 25 mg/mL, preferably from 0.5 to 15 mg/mL, (ii) at least one preservative comprising one or more parabens in an amount from 0.001 to 1% by weight of the formulation and (iii) a buffer comprising citric acid and a phosphate salt, the formulation having a pH of from 6.0 to 8.0, preferably a pH in the range of 6.5 to 8.0.

The formulation may comprise (i) an oligonucleotide which is cobitolimod in an amount from 0.1 to 25 mg/mL, preferably from 0.5 to 15 mg/mL, (ii) at least one preservative comprising Na-methylparaben and/or Na-propylparaben, wherein the at least one preservative is present in an amount from 0.001 to 1% by weight of the formulation and (iii) a buffer comprising citric acid and disodium hydrogenphosphate (NazHPOa), the formulation having a pH of from 6.0 to 8.0, preferably a pH in the range of 6.5 to 8.0.

The formulation may comprise (i) an oligonucleotide which is cobitolimod in an amount from 0.1 to 25 mg/mL, preferably from 0.5 to 15 mg/mL, (ii) a combination of Na-methylparaben and Na-propylparaben in an amount from 0.001 to 1% by weight of the formulation and (iii) a buffer comprising citric acid and disodium hydrogenphosphate (Na₂HPO₄), the formulation having a pH of from 6.0 to 8.0, preferably a pH in the range of 6.5 to 8.0.

### Pre-filled enema

The invention also provides a pre-filled enema suitable for self-administration, said pre-filled enema comprising:
(a) a container; and within the container
(b) the formulation as described herein.

Typically, the pre-filled enema is suitable for self-administration rectally. Thus, typically the enema may be administered by the patient themselves, without any specialist medical equipment or knowledge.

The skilled person would be well aware of various possible enema containers that may be used for rectal administration. Typically, such containers are plastic containers.

Typically, the container comprises a squeezable bottle. For instance, the bottle may be made from a material which is easily compressed by hand, thereby forcing the formulation out of the container. The shape of the bottle may also facilitate compression. For instance, the squeezable bottle may be a concertina-shaped bottle. In such bottles, the folds permit easy compression of the bottle and effective expulsion of the formulation inside.

Typically, the bottle is formed from polyethylene. Preferably the bottle is formed from low-density polyethylene. The material the bottle is formed from may also comprise small amounts of other additives, e.g opacifiers such as titanium dioxide, or colorants.

The bottle is typically of a size suitable to hold a therapeutically useful amount of the formulation. Typically, the bottle will hold a volume of 50 mL of the formulation. The bottle typically has a height of between 4 and 12 cm, preferably from 6 to 9 cm, typically around 7 to 9 cm. The bottle typically has a diameter of from 3 to 6 cm, preferably from 4 to 5 cm.

The container typically comprises an applicator suitable for dispensing the formulation. Thus, when the bottle is compressed, the formulation is expelled from the container *via* the applicator. Thus, typically, the applicator is formed from a material which is soft, so as not to cause injury, but also which does not easily break apart. The applicator may be formed from plastic. Typically, the applicator is formed from polyethylene, preferably from low density polyethylene. The material the applicator is formed from may also comprise small amounts of other additives, e.g opacifiers such as titanium dioxide, colorants or other plastic materials in low quantities such as polyamide and polyethylene-polyvinylacetate copolymer.

Typically, the applicator comprises a tip suitable for insertion into the rectum. The tip is from 4 to 15 cm long, typically from 4 to 10 cm long, preferably 5 to 6 cm long. The tip of the applicator may be lubricated for ease of administration.

Typically, the container of the pre-filled enema comprises a squeezable bottle and an applicator suitable for dispensing the formulation, wherein the applicator comprises a tip suitable for insertion into the rectum, wherein both the bottle and applicator are formed from polyethylene. The container of the pre-filled enema may comprise a concertina-shaped bottle and an applicator suitable for dispensing the formulation, wherein the applicator comprises a tip suitable for insertion into the rectum, wherein both the bottle and applicator are formed from polyethylene. Preferably both the bottle and applicator are formed from low density polyethylene.

The applicator may comprise a check valve. Typically, the check valve comprises a spring and a spring holder. The check valve regulates the flow of the formulation out of the container, and only allows the formulation to flow out of the container via the applicator if sufficient pressure is applied to the container. Typically, when the pressure is relieved, the check valve closes thereby preventing reflux of the formulation back into the bottle.

The pre-filled enema may further comprise a cap, which covers and protects the applicator until the pre-filled enema is used.

The amount of formulation within the container is not particularly limited and may be any amount that delivers a therapeutic benefit to the patient. Typically, the amount of the formulation within the container is no more than 60 mL, for instance from 20 to 60 mL, preferably from 30 to 60 mL, more preferably from 40 to 60 mL. The amount of the formulation within the container may be about 50 mL. In some instances, the amount of the formulation within the container may be about 30 mL.

The pre-filled enema typically comprises a single unit dosage of the oligonucleotide, preferably cobitolimod. The term "single unit dosage" refers to the formulation of the oligonucleotide apportioned into a dose comprising the amount of the compound specified.

Typically, the pre-filled enema comprises from 100 to 650 mg of the oligonucleotide, preferably cobitolimod. For instance, the pre-filled enema may comprise a dose of from 100 to 350 mg of the oligonucleotide, preferably cobitolimod. The pre-filled enema may comprise a dose of from 350 to 650 mg of the oligonucleotide, preferably cobitolimod. The pre-filled enema may comprise a dose of from 150 to 350 mg of the oligonucleotide, preferably cobitolimod. The pre-filled enema may comprise a dose of about 125 mg of the oligonucleotide, preferably cobitolimod. The pre-filled enema may comprise a dose of about 250 mg of the oligonucleotide, preferably cobitolimod. The pre-filled enema may comprise a dose of about 500 mg of the oligonucleotide, preferably cobitolimod.

The invention therefore provides a pre-filled enema suitable for self-administration, said pre-filled enema comprising:
(a) a container comprising a squeezable bottle and an applicator suitable for dispensing the formulation, wherein the applicator comprises a tip suitable for insertion into the rectum, preferably wherein the bottle and applicator are formed from plastic, preferably wherein the applicator further comprises a check valve;
(b) a formulation suitable for rectal administration comprising an oligonucleotide, preferably cobitolimod, (ii) at least one preservative comprising one or more parabens and (iii) a buffer comprising citric acid and a phosphate salt.

For instance, the pre-filled enema may comprise:
(a) a container comprising a concertina-shaped bottle and an applicator suitable for dispensing the formulation, wherein the applicator comprises a tip suitable for insertion into the rectum, wherein the bottle and applicator are formed from polyethylene, preferably wherein the applicator comprises a check valve; and within the container
(b) a formulation suitable for rectal administration comprising (i) an oligonucleotide which is cobitolimod in an amount from 0.1 to 25 mg/mL, preferably from 0.5 to 15 mg/mL, (ii) at least one preservative comprising one or more parabens in an amount from 0.001 to 1% by weight of the formulation and (iii) a buffer comprising citric acid and a phosphate salt, the formulation having a pH of from 6.0 to 8.0, preferably a pH in the range of 6.5 to 8.0.

The pre-filled enema may comprise:
(a) a container comprising a concertina-shaped bottle and an applicator suitable for dispensing the formulation, wherein the applicator comprises a tip suitable for insertion into the rectum, wherein both the bottle and applicator are formed from polyethylene, preferably low density polyethylene, preferably wherein the applicator comprises a check valve; and within the container
(b) a formulation suitable for rectal administration comprising (i) an oligonucleotide which is cobitolimod in an amount from 0.1 to 25 mg/mL, preferably from 0.5 to 15 mg/mL, (ii) at least one preservative comprising Na-methylparaben and/or Na-propylparaben, wherein the at least one preservative is present in an amount from 0.001 to 1% by weight of the formulation and (iii) a buffer comprising citric acid and disodium hydrogenphosphate (Na₂HPO₄), the formulation having a pH of from 6.0 to 8.0, preferably a pH in the range of 6.5 to 8.0.

The pre-filled enema may comprise:
(a) a container comprising a concertina-shaped bottle and an applicator suitable for dispensing the formulation, wherein the applicator comprises a tip suitable for insertion into the rectum, wherein both the bottle and applicator are formed from polyethylene, preferably low density polyethylene, preferably wherein the applicator comprises a check valve; and within the container
(b) a formulation suitable for rectal administration comprising (i) an oligonucleotide which is cobitolimod in an amount from 0.1 to 25 mg/mL, preferably from 0.5 to 15 mg/mL, (ii) a combination of Na-methylparaben and Na-propylparaben in an amount from 0.001 to 1% by weight of the formulation and (iii) a buffer comprising citric acid and disodium hydrogenphosphate (Na₂HPO₄), the formulation having a pH of from 6.0 to 8.0, preferably a pH in the range of 6.5 to 8.0.

### Pack

The invention also provides a pack comprising one or more pre-filled enemas as described herein.

Typically, the pack comprises a packaging gas. The packaging gas may be any gas which helps to prevent degradation of the formulation. Preferably the packaging gas is nitrogen.

The pack is usually a blister pack, comprising one or more pockets in which the one or more pre-filled enemas are sealed. Typically, each pre-filled enema is sealed in a single pocket i.e. each pre-filled enema is individually packaged. The skilled person would be well aware of suitable materials for blister packaging which act as a barrier to gases in the external environment. Blister packaging typically comprises a blister sealed with a lidding foil. Typical materials for the blister include plastics such as polyethylene terephthalate (PET), polyethylene (PE), ethylene vinyl alcohol (EVOH). The lidding foil typically comprises a metal barrier layer, for instance an aluminium barrier layer, and may comprise further layers to strengthen the packaging such as plastic and/or paper layers. For instance, the blister packaging may comprise a blister made from three layers such as a layer of PET/PE, a layer of EVOH (ethylene vinyl alcohol), and a layer of PE. The lidding foil may comprise layers of paper, aluminium and PE.

The one or more pockets typically contain a packaging gas, preferably nitrogen.

### Medical use

The invention also provides a formulation as described herein for use in the treatment of an inflammatory bowel disease. Preferably the inflammatory bowel disease is ulcerative colitis or Crohn's disease.

The formulation is typically administered topically to mucosal membranes. Preferably, the formulation is administered rectally. The formulation may be administered rectally using the pre-filled enema as described herein. Typically, the formulation is self-administered using the pre-filled enema as described herein.

The invention also provides the formulation as described herein for use in a method of treating an inflammatory bowel disease in a subject, the method comprising rectally administering to said subject the formulation. Preferably the inflammatory bowel disease is ulcerative colitis or Crohn's disease.

As used herein, the term "subject" refers to a human subject/patient. The terms "subject" and "patient" are used interchangeably herein.

Typically, the formulation is administered using the pre-filled enema as described herein. Preferably the formulation is self-administered by the subject using the pre-filled enema as described herein.

Typically, the subject has not been subjected to colonic cleaning prior to said administration. Thus, for any subject as described herein, and for any formulation or pre-filled-enema as described herein, typically, the subject has not been subjected to colonic cleaning prior to said administration. Thus, one advantage of the pre-filled enema of the invention is that complex preparation steps are not required for effective administration of the formulation.

As used herein, reference to a subject "which has not been subjected to colonic cleaning" is intended to define a subject which has not been subject to colonic cleaning in a time period prior to the administration of the formulation to reduce the amount of faecal matter in the colon treated with the oligonucleotide. Thus, typically the subject has not been subjected to colonic cleaning for 1 hour prior to the treatment with the formulation, typically for 4 hours prior to the treatment with the formulation, preferably for 8 hours prior to the treatment with the formulation, more preferably for 12 hours prior to the treatment with the formulation, more preferably for 24 hours prior to the treatment with the formulation, most preferably for 48 hours prior to the treatment with the formulation.

Typically, said colonic cleaning can be effected by any method known in the art, for example administration of a laxative.

Typically, said subject has luminal faecal material. Typically, said subject has faecal material which coats or is proximal to the colonic epithelial cells treated with the formulation comprising the oligonucleotide. Said subject may have faecal material in the lumen which is not directly in contact with the colonic epithelial cells treated with the formulation comprising the oligonucleotide. Said subject may have faecal material in the lumen which is coats or is proximal to the colonic epithelial cells treated with the formulation comprising the oligonucleotide and faecal material in the lumen which is not directly in contact with the colonic epithelial cells treated with the oligonucleotide. More preferably, the amount of said faecal material is the normal amount which would be expected in a patient which had never been subjected to colonic cleaning.

The invention also provides the use of a formulation as described herein for the manufacture of a medicament for treatment of inflammatory bowel disease. Preferably the inflammatory bowel disease is ulcerative colitis or Crohn's disease.

As used herein, the term inflammatory bowel disease (IBD) refers to a group of inflammatory conditions of the colon and the gastrointestinal tract. The major types of IBD are ulcerative colitis (UC) and Crohn's disease. The main difference between UC and Crohn's disease is the location and nature of the inflammatory changes. Crohn's disease can affect any part of the gastrointestinal tract, from mouth to anus, while UC is restricted to the colon and the rectum. In some cases, a definitive diagnosis of either Crohn's disease or UC cannot be made due to idiosyncrasies in the presentation. In these cases a diagnosis of indeterminate colitis may be made. Other forms of IBD include, but are not limited to, collagenous colitis, lymphocytic colitis, ischaemic colitis, diversion colitis, Behçet's disease and indeterminate colitis.

Typically, the inflammatory bowel disease is ulcerative colitis (UC).

The disease ulcerative colitis (UC) is well known to one skilled in the art. Ulcerative colitis treated in accordance with the present invention may involve treatment of ulcerative proctitis, distal or left-sided colitis, extensive colitis, pancolitis and pouchitis. Typically, the patient is suffering from left-sided ulcerative colitis.

Patients with UC typically present with a spectrum of disease severity ranging from remission to severely active. Clinical assessment can be used to classify UC patients into 4 disease activity subgroups as defined in D'Haens, Gastroenterology 2007; 132: 763-786: (1) remission (≤2 or 3 stools/ day, without the presence of blood and/or pus in the stools, with no systemic symptoms); (2) mildly active disease (3 or 4 stools/day and/or presence of blood and/or pus in the stools less than daily, with no systemic symptoms of fever or weight loss); (3) moderately active disease (>4 stools/day and/or daily presence of blood and/or pus) with minimal systemic symptoms; and (4) severely active disease (>6 bloody stools/day, and evidence of toxicity, as demonstrated by fever, tachycardia, anemia, or an erythrocyte sedimentation rate ESR). Clinical assessments can be classified with other scores e.g. Mayo Score, or variations thereof, or other indices.

Typically, the patient is suffering from moderate to severe UC. Preferably, the patient is suffering from moderate to severe UC as defined above. For instance, the patient may be suffering from moderate to severe left-sided UC.

As used herein, the words "treatment" and "treating" are to be understood as embracing treatment and/or amelioration and/or prevention of or reduction in aggravation/worsening of symptoms of a disease or condition as well as treatment of the cause of the disease or condition, and may include reversing, reducing, or arresting the symptoms, clinical signs, and underlying pathology of a condition in a manner to improve or stabilise a subject's condition.

In particular in the context of ulcerative colitis, "treating" typically refers to inducing response or remission in a patient having active ulcerative colitis. Thus, typically, the oligonucleotide is for inducing response or remission of active ulcerative colitis in a patient. Inducing response means improving the condition of a patient by e.g. reducing and/or arresting the symptoms and clinical signs of the active disease. Inducing remission means transitioning a patient from a state where they are considered to be in an active stage of the disease to a state where they are considered to be in remission. "Treating" may refer to maintaining a patient in remission.

Induction of response or remission in UC patients is typically assessed by one or more of endoscopy, histology, patient recorded outcomes and quality of life outcomes. Thus, reference to induction of response or remission includes induction of one or more of endoscopic remission, endoscopic response, histological remission, histological response, response or remission as determined by physician or by patient recorded outcomes, and response or remission as determined by quality of life. This can typically be assessed by reference to one or more standard indices.

Typically, ulcerative colitis is chronic active ulcerative colitis, i.e. the patient is suffering from chronic ulcerative colitis which is in an active phase.

As used herein, the term "chronic active ulcerative colitis" refers to patients with ulcerative colitis that is both active and chronic. Active ulcerative colitis is typically as defined herein, i.e. the patient is not in remission. Ulcerative colitis is typically a chronic (long-term) condition which cycles between active and less active phases. As the skilled person would understand, a patient suffering from chronic ulcerative colitis goes through active phases ("flare ups" or relapses) with more extreme symptoms interspersed with periods where the patient experiences milder symptoms or is in remission. Thus, the term "chronic active UC" typically refers to a patient with chronic UC who is in an active disease state.

The patient may be suffering from chronic active moderate to severe UC. The patient may be suffering from chronic active moderate to severe left-sided UC.

Preferably, reference herein to "treating" refers to inducing response or remission in a patient having chronic active ulcerative colitis. Thus, typically, the oligonucleotide is for inducing response or remission of chronic active ulcerative colitis in a patient.

Induction of response or remission in UC patients may be determined in accordance with one or more standard disease indices. Typical disease indices include but not limited to the ones mentioned below; (i) disease activity determined by clinical and biochemical disease activity, (ii) disease activity determined by endoscopic disease activity, (iii) disease activity determined by composite clinical and endoscopic disease activity indices, (iv) quality of life, (v) histologic disease activity. These indices are discussed in D'Haens (ibid).

Indices based on disease activity determined by clinical and biochemical disease activity include the Truelove and Witts Severity Index; Powell-Tuck (St. Mark's) Index; Clinical Activity (Rachmilewitz) Index; Activity (Seo) Index; Physician Global Assessment; Lichtiger (Modified Truelove and Witts Severity) Index; Investigators Global Evaluation; Simple Clinical Colitis Activity Index; Improvement Based on Individual Symptom Scores; Ulcerative Colitis Clinical Score; and Patient-defined remission. These indices are discussed in D'Haens (ibid).

Indices based on disease activity determined by endoscopic disease activity include the Truelove and Witts Sigmoidoscopic Assessment; Baron score; Powell-Tuck Sigmoidoscopic Assessment; Endoscopic (Rachmilewitz Endoscopic) Index; Sigmoidoscopic Index; Sigmoidoscopic Inflammation Grade Score; Mayo Score Flexible Proctosigmoidoscopy Assessment; Sutherland Mucosal Appearance Assessment; and Modified Baron Score. These indices are discussed in D'Haens (ibid).

Indices based on disease activity determined by composite clinical and endoscopic disease activity indices include the Mayo Score (Mayo Clinic Score/Disease Activity Index); Modified Mayo Score and Sutherland Index (Disease Activity Index/UC Disease Activity Index). Mayo Score and Sutherland Index are discussed in D'Haens (ibid).

Indices based on quality of life include the Rating Form of IBD Patient Concerns; and the Inflammatory Bowel Disease Questionnaire (IBDQ). These indices are discussed in D'Haens (ibid).

Indices based on histologic disease activity include those discussed in D'Haens (ibid) such as Geboes Index and Riley Index and further indices such as Nancy Histological Index and Robarts Histopathology Index.

The preferred index for assessing UC patients in clinical drug development is the Mayo Score and Modified Mayo Scores as described in the guidelines from the regulatory agencies European Medicinal Agency (EMA) and Food and Drug Administration (FDA) (Guidance Document: Ulcerative Colitis: Clinical Trial Endpoints Guidance for Industry, Aug 2016, docket no. FDA-2016-D-2319; EMA, Guideline on the development of new medicinal products for the treatment of Ulcerative Colitis 21 July 2016).

The Clinical Activity (Rachmilewitz) Index is an index taking into account 7 variables: number of stools, blood in stools, investigator's global assessment of symptomatic state, abdominal pain or cramps, temperature due to colitis, extraintestinal manifestations, and laboratory findings. This is discussed further in D'Haens (ibid) and Rachmilewitz D., BMJ 1989; 298: 82-86. Determination of the Clinical Activity (Rachmilewitz) Index produces a score for a patient ranging from 0 to 29 points (higher scores meaning more severe disease).

Clinical remission may be considered as a Clinical Activity (Rachmilewitz) Index score ≤4 points. Response as determined by the Clinical Activity (Rachmilewitz) Index means the patient has a lower score after treatment than before treatment.

The Mayo Score is an index taking into account 4 items: stool frequency, rectal bleeding, findings of endoscopy, and Physician's Global Assessment (PGA). This is discussed further in D'Haens (ibid) and Schroeder KW et al, N Engl J Med 1987; 317: 1625-1629. Determination of the Mayo Score produces a score ranging from 0 to 12 points (higher scores meaning more severe disease). In addition to the four specific items, a patient's functional assessment is also measured that is not meant to be included in the 12-point index calculation but should be used as a measure of general well-being when determining the PGA score.

Mayo scoring for each of the 4 items is determined as set out in the Table below.

| Score | Stool frequency^{b} | Rectal Bleeding^{c} | Physician's global assessment^{d} | Colonoscopy/sigmoidoscopy finding |
|---|---|---|---|---|
| 0 | Normal number of stools for this patient | No blood seen | Normal or no disease | Normal or inactive disease |
| 1 | 1 to 2 stools more than normal | Streaks of blood with stool less than half of the time | Mild disease | Mild disease (erythema, decreased vascular pattern, mild friability) |
| 2 | 3 to 4 stools more than normal | Obvious blood with stool most of the time | Moderate disease | Moderate disease (marked erythema, lack of vascular pattern, friability, erosions) |
| 3 | 5 or more stools more than normal | Blood alone passed | Severe disease | Severe disease (spontaneous bleeding, ulceration) |

| | | | | |
|---|---|---|---|---|
| ^{b} Each patient serves as his or her own control to establish the degree of abnormality of the stool frequency. ^{c} The daily bleeding score represents the most severe bleeding of the day. ^{d} The physician's global assessment acknowledges the 3 other criteria, the patient's daily record of abdominal discomfort and general sense of well-being, and other observations, such as physical findings and the patient's performance status. | | | | |

Remission according to the Mayo Score may be defined as complete resolution of (1) stool frequency (normal stool frequency), (2) rectal bleeding (no rectal bleeding), (3) patient's functional assessment score (generally well), (4) endoscopy findings (normal), and a PGA score of 0. Response as determined by Mayo Score typically requires improvement (a minimum 1-point decrease from baseline) in the PGA score and improvement in at least one other clinical assessment (stool frequency, rectal bleeding, patient's functional assessment, endoscopy findings) and no worsening in any other clinical assessment.

Alternatively, clinical remission may be defined as a Mayo Score of 0 and clinical improvement (response) as a decrease from baseline in the Mayo Score ≥3 points.

Alternatively, clinical remission may be defined as a Mayo Score of 0 and clinical improvement (response) as a decrease from baseline in the Mayo Score ≥3 points (or a decrease of ≥2 points if the baseline Mayo Score was ≤3 points).

Alternatively, remission as determined by Mayo Score may be defined as requiring
subscores of 0 for both sigmoidoscopy and rectal bleeding and a score of 0 or 1 for stool frequency and PGA subscores. Response may be defined as a decrease from baseline in
the Mayo Score ≥3 points; clinical response may be defined as a decrease from baseline in the Mayo Score (without the endoscopy subscore, also known as a Partial Mayo Score)
≥2 points, and endoscopic response may be defined as a decrease from baseline in the endoscopic subscore ≥1 point.

Alternatively, clinical remission may be defined as a total Mayo score of ≤2 points with no
individual subscore >1 point, clinical response may be defined as a decrease from baseline in the total Mayo score ≥3 points and ≥30% and a decrease in the rectal bleeding subscore
≥1 point or an absolute rectal bleeding subscore of 0 or 1, and mucosal healing may be defined as an absolute endoscopy subscore of 0 or 1.

In one embodiment, patients having active ulcerative colitis have a Mayo Score >2. Patients who are in a remission phase of ulcerative colitis typically have a Mayo Score ≤2.

Modified Mayo Score is related to the Mayo Score, which is defined above. Modified Mayo Score is any modification to the Mayo Score. Central reading for evaluation of the endoscopy assessment is now standard in drug development. The recommendations from the FDA for the modified Mayo score are that the colonoscopy/sigmoidoscopy scoring takes less account of friability in grade 1. In addition, the FDA recommends to remove PGA from the score, as it includes a degree of subjective evaluation. Those two modifications are usually what is referred to as the Modified Mayo Score

Remission and response values for the Modified Mayo Score are defined in clinical development and may vary slightly amongst the different drugs. Further information may be found in "Ulcerative Colitis: Clinical Trial Endpoints Guidance for Industry" found at
http://www.fda.gov/downloads/Drugs/GuidanceComplianceRegulatoryInformation/Guidances/UCM515 143.pdf

Induction of remission of UC may be in accordance with the criteria set out in S. P. L. Travis, Aliment Pharmacol Ther 2011; 34: 113-124, i.e. complete cessation of rectal bleeding, urgency and increased stool frequency, preferably confirmed by endoscopic mucosal healing.

Alternatively, induction of response or remission may be in accordance with the criteria set out in E.F. Stange, Journal of Crohn's and Colitis (2008) 2, 1-23; S.P.L. Travis, Journal of Crohn's and Colitis (2008) 2, 24-62; K Geboes, Gut 2000; 47: 404-409.

Induction of response or remission in Crohn's disease patients may be determined in accordance with one or more standard disease indices. Typical indices include the Crohn's Disease Activity Index (CDAI). The CDAI is discussed in Love, "Pharmacotherapy for Moderate to Severe Inflammatory Bowel Disease: Evolving Strategies", Am J Manag Care. 2016;22:S39-S50; Peyrin-Biroulet et al "Defining disease severity in inflammatory bowel diseases: current and future directions" Clin Gastroenterol Hepatol. 2015; pii: S1542-3565(15)00787-00789. doi: 10.1016/j.cgh.2015.06.001; and Ungar et al "Advances in the development of new biologics in inflammatory bowel disease", Annals of Gastroenterology (2016) 29, 243-248. Alternative indices for assessing Crohn's disease patients include the Harvey-Bradshaw index and the Inflammatory Bowel Disease Questionnaire.

CDAI is a composite score taking into account a large number of symptoms associated with Crohn's disease, including number of liquid or soft stools; abdominal pain; general well-being; presence of complications (the presence of joint pains (arthralgia) or frank arthritis; inflammation of the iris or uveitis; presence of erythema nodosum, pyoderma gangrenosum, or aphthous ulcers; anal fissures, fistulae or abscesses; other fistulae; fever during the previous week); use of lomotil or opiates for diarrhea; presence of an abdominal mass; hematocrit value; and percentage deviation from standard weight. Clinical remission according to the CDAI is typically indicated by a score of <150.

The subject treated in accordance with the present invention is typically refractory or responds insufficiently or is intolerant to anti-inflammatory therapy and/or demonstrates or has previously demonstrated an inadequate response, loss of response, or intolerance to at least one immunomodulator, TNF-α inhibitor, anti-integrin, JAK inhibitors or IL-12/IL-23 inhibitor (also known as advanced therapies). The subject treated in accordance with the present invention is typically refractory or responds insufficiently or is intolerant to anti-inflammatory therapy. The subject treated in accordance with the present invention is typically refractory or responds insufficiently or is intolerant to at least one immunomodulator. The subject treated in accordance with the present invention is typically refractory or responds insufficiently or is intolerant to at least one TNF-α inhibitor.

Thus, typically, the subject has previously received or is currently receiving anti-inflammatory therapy, preferably anti-inflammatory therapy for UC and/or immunomodulatory, TNF-α inhibitor or anti-integrin therapy, preferably such therapy for UC.

Anti-inflammatory therapies for UC are discussed herein and typically include oral corticosteroids, and glucocorticosteroids (GCS), sulfasalazine and 5-ASA. Thus, the subject treated in accordance with the present invention is typically refractory or responds insufficiently or is intolerant to GCS, sulfasalazine and/or 5-ASA. Typically, the subject treated in accordance with the present invention is refractory or responds insufficiently or is intolerant to oral corticosteroids.

Immunomodulators, TNF-α inhibitors and anti-integrins are discussed herein and typically include azathioprine, 6-mercaptopurine and biologicals including the TNF-α inhibitors infliximab and biosimilars and derivatives thereof, golimumab and biosimilars and derivatives thereof, adalimumab and biosimilars and derivatives thereof and anti-integrins vedolizumab and biosimilars and derivatives thereof. Thus, the subject treated in accordance with the present invention is typically refractory or responds insufficiently or is intolerant to azathioprine, 6-mercaptopurine, infliximab and biosimilars and derivatives thereof, golimumab and biosimilars and derivatives thereof, adalimumab and biosimilars and derivatives thereof and anti-integrin vedolizumab and biosimilars and derivatives thereof. Typically, the subject treated in accordance with the present invention is refractory or responds insufficiently or is intolerant to azathioprine and/or 6-mercaptopurine. Typically, the subject treated in accordance with the present invention is refractory or responds insufficiently or is intolerant to infliximab and/or adalimumab.

The subject treated in accordance with the present invention may be refractory or respond insufficiently or is intolerant to JAK inhibitors (for instance tofacitinib). The subject treated in accordance with the present invention may be refractory or respond insufficiently or is intolerant to IL-12/IL23 inhibitor (for instance ustekinumab).

A refractory disease or disease that responds insufficiently to therapy is typically a disease where signs and symptoms of active disease persist despite a history of at least one course of therapy, for instance anti-inflammatory therapy or immunomodulatory therapy, in the context of the present invention. Typically, in the context of treatment of UC, signs and symptoms of active disease persist despite a history of two or more courses of anti-inflammatory therapy or immunomodulatory therapy. A typical course of treatment with anti-inflammatory or immunomodulatory therapy for UC would be well understood by a person skilled in the art, and would typically involve a sufficient number of doses at sufficient dosage to induce remission in a typical patient.

Intolerance to therapy, for instance anti-inflammatory therapy or immunomodulatory therapy including the so-called advanced therapies, in the context of the present invention, means that the therapy has caused side effects in the subject that are not tolerated, e.g. that typically lead to discontinuation of therapy.

Typically, the subject has previously received or is currently receiving Aminosalicylic acid (5-ASA), preferably 5-ASA therapy for UC.

Typically, the subject has previously received or is currently receiving oral Glucocorticosteroids (GCS), preferably oral GCS therapy for UC.

Typically, the subject who is refractory or responds insufficiently or is intolerant to anti-inflammatory therapy shows or has previously shown an inadequate response to, or loss of response to (i.e. is refractory to) or intolerance of rectal, oral, and/or parenteral GCS treatment (including no GCS treatment due to earlier side effect).

Typically, the subject who is refractory or responds insufficiently or is intolerant to anti-inflammatory therapy has a history of or current status of an inadequate response (e.g. steroid refractory) to, OR steroid dependency, OR loss of response to, OR intolerance of GCS treatment. The steroids/GCS will typically have been received by the subject in the course of treating ulcerative colitis.

Steroid-refractory typically refers to a subject lacking a meaningful clinical response, i.e. showing signs and symptoms of persistently active ulcerative colitis, despite a history of at least one course of steroid treatment, for instance an induction regimen that included a dose equivalent to prednisone 40-60 mg daily over a period of 30 days for oral administration or over a period of 7 to 10 days for intravenous (IV) administration.

Steroid dependence typically refers to a patient who is either unable to reduce steroids below the equivalent of prednisolone 10 mg/d within 3 months of starting steroids, without recurrent active ulcerative colitis, or who has a relapse within 3 months of stopping steroids.

Intolerance of GCS treatment typically means the subject has experienced side effects not tolerated by the subject following GCS treatment, such as but not limited to Cushing's syndrome, osteopenia/osteoporosis, hyperglycemia, insomnia, or infection.

Typically, the subject who is refractory or responds insufficiently or is intolerant to an immunomodulator shows or has previously shown an inadequate response to, or loss of response to (i.e. is refractory to) or intolerance of an immunomodulator. For instance, the subject who is refractory or responds insufficiently or is intolerant to a TNF-α inhibitor shows or has previously shown an inadequate response to, or loss of response to (i.e. is refractory to) or intolerance of a TNF-α inhibitor.

An inadequate response, or loss of response to an immunomodulator typically means signs and symptoms of active ulcerative colitis persist despite previous treatment with at least one immunomodulator, for instance one 8 Week regimen of oral azathioprine (>_1.5 mg/kg) or 6-mercaptopurine (>_0.75 mg/kg).

Intolerance to an immunomodulator typically means the subject has experienced nausea/vomiting, abdominal pain, pancreatitis, liver function test (LFT) abnormalities, lymphopenia, Thiopurine Methyltransferase (TPMT) genetic mutation, or infection or other side effects after receiving an immunomodulator.

An inadequate response, or loss of response to a TNF-α inhibitor means signs and symptoms of active ulcerative colitis persist despite previous treatment with at least one TNF-α inhibitor, such as 4-Week induction regimen (or doses as recommended according to the current labels) of infliximab (5 mg/kg (IV), 2 doses at least 2 weeks apart) or a biosimilar or derivative thereof; golimumab (200 /100 mg (SC), 2 doses at least 2 weeks apart) or a biosimilar or derivative thereof; or adalimumab (160/80 mg (SC), 2 doses at least 2 weeks apart) or a biosimilar or derivative thereof or recurrence of symptoms during maintenance dosing following prior clinical benefit.

Intolerance to a TNF-α inhibitor means an infusion-related reaction, demyelination, congestive heart failure, infection or other side effects following receipt of a TNF-α inhibitor.

An inadequate response, or loss of response to an anti-integrin means signs and symptoms of active ulcerative colitis persist despite previous treatment with an anti-integrin, for instance at least 10 weeks regimen of vedolizumab 300 mg (IV) or a biosimilar or derivative thereof, or as recommended in the current label, or recurrence of symptoms during maintenance dosing following prior clinical benefit.

Typically, the subject has been diagnosed with left-sided ulcerative colitis, i.e. distal colitis, including proctosigmoiditis. For instance, the subject may have been diagnosed with moderate to severe left-sided ulcerative colitis.

Typically, said subject is elective for colectomy.

As used herein, the term "colectomy" refers to surgical resection of any extent of the large intestine (colon). Herein, colectomy includes, but is not limited to, right hemicolectomy, left hemicolectomy, extended hemicolectomy, transverse colectomy, sigmoidectomy, proctosigmoidectomy, Hartmann operation, "double-barrel" or Mikulicz colostomy, total colectomy (also known as Lane's Operation), total procto-colectomy and subtotal colectomy. As used herein, the phrase "elective for colectomy" refers to a subject who may choose to undergo the procedure of non-emergency colectomy based on physician and surgeon assessment. Subjects elective for colectomy may be, but are not limited to, subjects refractory to available therapy (for ulcerative colitis) or intolerant of available therapy (for ulcerative colitis). This differs from emergency colectomy, which is an acute intervention for subjects with acute illnesses or injuries and who require immediate medical attention. The phrase also includes subjects that are elected for colectomy.

The invention also provides the formulation as described herein or the pre-filled enema as described herein for use in preventing the recurrence of active ulcerative colitis in a patient.

Thus, the formulation may be for use as a maintenance therapy. In the context of this aspect of the present invention, reference to preventing the recurrence of active ulcerative colitis is intended to refer to preventing recurrence of the active phase of the disease, i.e. maintaining a patient in remission and providing a maintenance therapy. Thus, the present invention relates to the use of the formulation as described herein to prevent the recurrence of or relapse into the active phase of ulcerative colitis. The formulation as herein above can be used as maintenance therapy to prevent the recurrence of or relapse into symptoms associated with active ulcerative colitis, and/or to improve the patient's condition.

The patient may be any patient or subject as described herein. For instance, the patient may be suffering with any type of ulcerative colitis as described herein. Typically, the subject has been diagnosed with left-sided ulcerative colitis, i.e. distal colitis, including proctosigmoiditis.

Typically, the patient has previously been treated for active ulcerative colitis, for instance the patient may have previously received one or more therapeutic agents for the treatment of active ulcerative colitis. The one or more therapeutic agents may comprise the oligonucleotide of SEQ ID NO:2 as described herein. The one or more therapeutic agents may comprise any other therapeutic agent used to treat ulcerative colitis, as described herein.

The use of cobitolimod as a maintenance therapy is described in WO 2020/099585.

### EXAMPLES

### Example 1 - degradation studies

In the case of cobitolimod, the main impurity observed in the drug substance (and drug product) is the major product-related impurity N-3'PO/N-5'PO with the relative retention time (RRT) of 0.95-0.96 (using IP-RP HPLC). This impurity is formed by oxidation of one phosphothioate to the corresponding PO, i.e. one sulfur atom is substituted by an oxygen atom. The resulting impurity is closer in structure to regular endogenous DNA compared to cobitolimod.

During initial forced degradation studies the compound was subject to a freeze-drying cycle (5 repetitions) over 5 days without any detected degradation.

The thermal stability of cobitolimod at 50°C and 80°C was also tested over 4 days. Storage of samples at 50°C did not result in any degradation of the compound. At 80°C a significant degradation of approximately 4 area% was recorded after four days. LC/MS data show that at the high temperature there is an increase in levels of the PO impurity and also an increase in the depurination and pyrimidation products, although these are at low levels. These degradation products are typically expected for this type of oligonucleotide.

Further forced degradation studies by pH (acid, base), oxidation and photo-exposure have been performed on samples from the same lot.

Exposure to acid, peroxide and photo exposure gave rise to varying degrees of degradation.

No degradation was observed for the base exposed samples.

Acid and peroxide exposure gave rise to an increase in PO- and N-1 impurities and increase in the early eluting peaks. Also, photo exposure resulted in a slight increase of PO and N-1 impurities. As cobitolimod has a PO/PS internucleotide backbone, in the presence of hydrogen peroxide the PS convert to PO forming the PO impurity of cobitolimod.

### Analytical procedures:

### Purity, Impurities and Assay by IP-RP-HPLC

The purity, impurities and assay of the cobitolimod are determined by IP-RP HPLC. A nominal 0.5 mg/mL solution of sample is prepared in purified water. The analyte is eluted on a C18 analytical column, kept under temperature control, using a gradient solvent system. Detection is performed by UV at 260 nm.

### Purity and Impurities by IEX HPLC

The purity and impurities of cobitolimod can also be determined by IEX HPLC. A nominal 1 mg/mL solution of sample is prepared in purified water. The analyte is eluted on a DNAPac-100 analytical column, kept under temperature control, using a gradient buffer solution system. Detection is performed by UV at 260 nm.

### Conclusion

Cobitolimod shows sensitivity to degradation under certain storage conditions. In particular, elevated temperature, exposure to acid and oxidising agents all lead to degradation of cobitolimod. Minor degradation could be detected by exposure to light.

### Example 2 - Enema formulations and stability studies

Several batches of cobitolimod rectal solution have been manufactured, including a batch at 31mg/50mL (V2263), and batches at 250 mg/50 mL (V3009, V3080) and 500 mg/50 mL (V3045, V3081, V3112). Table 2 below summarizes the main differences between different batches.

**Table 1 - Components included in the enema batches**

| **Component** |
|---|
| Cobitolimod |
| Na-Methylparaben |
| Na-Propylparaben |
| Citric acid anhydrous |
| Disodium hydrogenphosphate dihydrate |
| Water, purified |

**Table 2 - Main differences between batches**

| **Parameter** | **Batch V2263** | **Batch V3009** | **Batch V3045** | **Comment** |
|---|---|---|---|---|
| Strength | 31 mg/50 mL | 250 mg/50 mL | 500 mg/50 mL | Increase strength of 500mg/50mL after results of clinical study CSUC-01/16 available |
| Container closure system | PE container with soft PVC applicator | PE container with soft PE applicator | PE container with soft PE applicator | Change of applicator for the prototype batches because of adsorption of paraben to PVC material |
| Manufacturing | Pilot plant 3.25 kg | Pilot plant 6.5 kg | Pilot plant 4.6 kg | None |
| Blistering and nitrogen flushing of blisters | No | Yes | Yes | Potentially reduce oxidation of API and water losses |

| | | | | |
|---|---|---|---|---|
| Batch V3080 has the same composition as V3009. Batches V3081 and V3112 have the same composition as V3045. | | | | |

Cobitolimod rectal solution is manufactured by dissolving the six components in Table 1 in water. All components are soluble in water. Sodium salts of the parabens and disodium phosphate dihydrate are more soluble in a neutral to basic pH. Citric acid anhydrous is the acidic component. Disodium phosphate dihydrate and citric acid anhydrous are the buffer system of the solution. The API is added as the last step to ensure that the solution is preserved and buffered.

### Stability data

### Stability summary and conclusion

Stability data for cobitolimod rectal solution at 250 mg/50 mL and 500 mg/50 mL are so far available for up to 24 months. Cobitolimod rectal solution at concentrations of at least 250 mg/50 mL is stable for up to 24 months when stored at 2-8°C (ambient relative humidity, RH) and 25°C (40% RH) and for up to 6 months when stored at 40°C (25% RH).

### Stability Protocol

Stability protocols of the indicative stability batch at 31 mg/50 mL is described in Table 3. This batch was manually manufactured using the initial enema bottle suggested by the manufacturer with a soft PVC applicator (V2263) and placed on stability (horizontal position). The enema bottles were not blistered.

**Table 3 - Program stability cobitolimod rectal solution, 31 mg/50 mL (batch V2263)**

| Storage temperature | Initial | 1 m | 3 m | 6 m | 9 m | 12 m |
|---|---|---|---|---|---|---|
| 25°C/40%RH | A, B, C | A | A | A | A | A, B |
| 40°C/25% RH | | A | A | A, B | - | - |

| | | | | | | |
|---|---|---|---|---|---|---|
| A: Appearance, colour of solution, pH, assay of API, purity of API, assay of preservatives, and purity of preservatives B: Microbiological quality C: Identity of API and identity of preservatives | | | | | | |

The stability program for the batch V3009 at 250 mg/50 mL (in final blistered container closure system: PE bottle and soft PE applicator) is described in Table 4.

**Table 4 - Program stability for cobitolimod rectal solution, 250 mg/50 mL (batch V3009)**

| **Storage temperature** | **Initial** | **1 m** | **3 m** | **6 m** | **9 m** | **12 m** | **18 m** | **24 m** | **36 m** | **48 m*** |
|---|---|---|---|---|---|---|---|---|---|---|
| 5°C | A, B, C | - | A | A | A | A, B | A | A, B | A, B | A, B |
| 25°C/40%RH | | A | A | A | A | A, B | A | A, B | A, B | - |
| 40°C/25% RH | | A | A | A, B | - | - | - | - | - | - |

| | | | | | | | | | | |
|---|---|---|---|---|---|---|---|---|---|---|
| A: Appearance, colour of solution, pH, assay of API, purity of API, assay of preservatives, and purity of preservatives B: Microbiological quality C: Identity of API, identity of preservatives * optional time point | | | | | | | | | | |

The stability program for the batch V3045 at 500 mg/50 mL (in final blistered container closure system: PE bottle and soft PE applicator) is described in Table 5.

**Table 5 - Program stability for cobitolimod rectal solution, 500 mg/50 mL (Batch V3045)**

| **Storage temperature** | **Initial** | **1 m** | **3 m** | **6 m** | **9 m** | **12 m** | **18 m** | **24 m** | **36 m** | **48 m*** |
|---|---|---|---|---|---|---|---|---|---|---|
| 5°C | A, B, C | - | A | A | A | A, B | A | A, B | A, B | A, B |
| 25°C/40%RH | | A | A | A | A | A, B | | A, B | A, B | - |
| 40°C/25% RH | | A | A | A, B | - | - | - | - | - | - |

| | | | | | | | | | | |
|---|---|---|---|---|---|---|---|---|---|---|
| A: Appearance, colour of solution, pH, assay of API, purity of API, assay of preservatives, and purity of preservatives B: Microbiological quality C: Identity of API, identity of preservatives * optional time point | | | | | | | | | | |

The stability program of the investigational medicinal product for batch V3080 and V3081 is described in Table 6.

**Table 6 - Stability program for cobitolimod rectal solution, 250 mg/50 mL (batch V3080) and 500 mg/50 mL (Batch V3081)**

| **Storage temperature** | **Initial** | **1 m** | **3 m** | **6 m** | **9 m** | **12 m** | **18 m** | **24 m** | **36 m*** |
|---|---|---|---|---|---|---|---|---|---|
| 5°C | A, B, C | - | A | A | A | A, B | A | A, B | A |
| 25°C/40%RH | | A | A | A, B | - | - | - | - | - |
| 40°C/25% RH | | A | A, B | - | - | - | - | - | - |

| | | | | | | | | | |
|---|---|---|---|---|---|---|---|---|---|
| A: Appearance, colour of solution, pH, assay of API, purity of API, assay of preservatives, and purity of preservatives B: Microbiological quality C: Identity of API, identity of preservatives * optional time point | | | | | | | | | |

### Example 3 - Stability studies

Extensive stability studies on different batches were carried out, as detailed above in the stability programs. Data are presented below.

### Stability data

Results from the stability program for the V3009 batch at 250 mg/50 mL are presented in Table 7 (5°C), Table 8 (25°C), and Table 9 (40°C).

Results from the stability program for the V3045 batch at 500 mg/50mL are presented in Table 10 (5°C), Table 11 (25°C) and Table 12 (40°C).

Results from the stability program for the V3080 batch at 250 mg/50 mL are presented in Table 13 (5°C), Table 14 (25°C) and Table 15 (40°C).

Results from the stability program for the V3081 batch at 500 mg/50 mL are presented in Table 16 (5°C), Table 17 (25°C) and Table 18 (40°C).

**Table 7. Stability cobitolimod rectal solution, 250 mg/50 mL, batch V3009 (5°C/ambient RH)**

| **Test** | **Acceptance criteria** | **Initial** | **3 m** | **6 m** | **9 m** | **12 m** | **18 m** | **24 m** |
|---|---|---|---|---|---|---|---|---|
| Appearance | Clear solution | Complies | Complies | Complies | Complies | Complies | Complies | Complies |
| Colour of solution | Colourless, NMT B9 | Complies | Complies | Complies | Complies | Complies | Complies | Complies |
| pH | 6.5-8.0 | Complies | Complies | Complies | Complies | Complies | Complies | Complies |
| Assay cobitolimod | 90-110% | 107 | 107 | 107 | 109 | 106 | 109 | 109 |
| Purity of cobitolimod (HPLC)* | | | | | | | | |
| - Sum of impurities | ≤10.0 area% | 4.1 | 4.6 | 4.2 | 4.6 | 3.9 | 4.2 | 4.6 |
| - Purity | ≥ 90.0 area% | 95.9 | 95.4 | 95.8 | 95.4 | 96.1 | 95.8 | 95.4 |

| Main degradation impurity | | | | | | | | |
|---|---|---|---|---|---|---|---|---|
| - RRT 0.96-0.98 (N-3'PO/N-5'PO) | ≤4.0 area% | 1.1 | 1.7 | 1.4 | 1.7 | 1.0 | 1.3 | 1.5 |
| Purity of preservatives | | | | | | | | |
| p-hydroxybenzoic acid | Report (mg/mL) | 0.01 | 0.01 | 0.01 | 0.01 | 0.01 | 0.02 | 0.02 |
| Microbial quality (Ph. Eur.5.1.4.) | | | | | | | | |
| TAMC | ≤10³ cfu/g | <10 | Nd | Nd | Nd | <1 | Nd | <1 |
| TYMC | ≤10² cfu/g | <10 | Nd | Nd | Nd | <1 | Nd | <1 |
| Evaporation, change of weight | ≤5.0% | - | 0.1 | 0.1 | 0.3 | 0.1 | 0.2 | 0.1 |

| | | | | | | | | |
|---|---|---|---|---|---|---|---|---|
| Nd: Not determined. * Reporting threshold: ≥0.15 area% | | | | | | | | |

**Table 8. Stability cobitolimod rectal solution, 250 mg/50 mL, batch V3009 (25°C/40%RH)**

| **Test** | **Acceptance criteria** | **Initial** | **1 m** | **3 m** | **6 m** | **9 m** | **12 m** | **18 m** | **24 m** |
|---|---|---|---|---|---|---|---|---|---|
| Appearance | Clear solution | Complies | Complies | Complies | Complies | Complies | Complies | Complies | Complies |
| Colour of solution | Colourless, NMT B9 | Complies | Complies | Complies | Complies | Complies | Complies | Complies | Complies |
| pH | 6.5-8.0 | Complies | Complies | Complies | Complies | Complies | Complies | Complies | Complies |
| Assay cobitolimod | 90-110% | 107 | 104 | 106 | 106 | 108 | 105 | 105 | 103 |
| Purity of cobitolimod (HPLC)* | | | | | | | | | |
| - Sum of impurities | ≤10.0 area% | 4.1 | 4.8 | 4.8 | 4.9 | 5.6 | 5.7 | 7.0 | 8.7 |
| - Purity | ≥ 90.0 area% | 95.9 | 95.2 | 95.2 | 95.1 | 94.5 | 94.3 | 93.0 | 91.3 |

| Main degradation impurity | | | | | | | | | |
|---|---|---|---|---|---|---|---|---|---|
| - RRT 0.96-0.98 (N-3'O/N-5'PO) | ≤4.0 area% | 1.1 | 1.8 | 2.1 | 2.0 | 2.6 | 2.2 | 2.9 | 3.4 |
| Purity of preservatives | | | | | | | | | |
| p-hydroxybenzoic acid | Report (mg/mL) | 0.01 | 0.01 | 0.02 | 0.04 | 0.05 | 0.07 | 0.11 | 0.13 |

| **Test** | **Acceptance criteria** | **Initial** | **1 m** | **3 m** | **6 m** | **9 m** | **12 m** | **18 m** | **24 m** |
|---|---|---|---|---|---|---|---|---|---|
| Microbial quality (Ph. Eur.5.1.4) | | | | | | | | | |
| TAMC | ≤10³ cfu/g | <10 | Nd | Nd | Nd | Nd | <1 | Nd | <1 |
| TYMC | ≤10² cfu/g | <10 | Nd | Nd | Nd | Nd | <1 | Nd | <1 |
| Evaporation, change of weight | ≤5.0% | - | -0.1 | -0.4 | -0.7 | -1.5 | -1.4 | -3.0 | -3.9 |

| | | | | | | | | | |
|---|---|---|---|---|---|---|---|---|---|
| Nd: Not determined. * Reporting threshold: ≥0.15 area% | | | | | | | | | |

**Table 9. Stability cobitolimod rectal solution, 250 mg/50 mL, batch V3009 (40°C/25%RH)**

| **Test** | **Acceptance criteria** | **Initial** | **1 m** | **3 m** | **6 m** |
|---|---|---|---|---|---|
| Appearance | Clear solution | Complies | Complies | Complies | Complies |
| Colour of solution | Colourless, NMT B9 | Complies | Complies | Complies | Complies |
| pH | 6.5-8.0 | Complies | Complies | Complies | Complies |
| Assay | | | | | |
| Cobitolimod | 90-110% | 107 | 104 | 104 | 102 |
| Purity of cobitolimod (HPLC)* | | | | | |
| Sum of impurities | ≤10.0 area% | 4.1 | 4.8 | 6.1 | 7.8 |
| Purity of cobitolimod | ≥ 90.0 area% | 95.9 | 95.2 | 93.9 | 92.2 |

| Main degradation impurity | | | | | |
|---|---|---|---|---|---|
| - RRT 0.96-0.98 (N-3'PO/N-5'PO) | ≤4.0 area% | 1.1 | 1.8 | 2.4 | 2.7 |
| Purity of preservatives | | | | | |
| p-hydroxybenzoic acid | Report (mg/mL) | 0.01 | 0.05 | 0.13 | 0.23 |
| Microbial quality (Ph. Eur.5.1.4) | | | | | |
| TAMC | ≤10³ cfu/g | <10 | Nd | Nd | 37 |
| TYMC | ≤10² cfu/g | <10 | Nd | Nd | <10 |
| Evaporation, change of weight | ≤5.0% | - | -0.6 | -1.7 | -3.2 |

| | | | | | |
|---|---|---|---|---|---|
| Nd: Not determined. * Reporting threshold: ≥0.15 area% | | | | | |

**Table 10. Stability cobitolimod rectal solution 500 mg/50 mL, batch V3045 (5°C/ambient RH)**

| **Test** | **Acceptance criteria** | **Initial** | **3 m** | **6 m** | **9 m** | **12 m** | **18 m** | **24 m** |
|---|---|---|---|---|---|---|---|---|
| Appearance | Clear solution | Complies | Complies | Complies | Complies | Complies | Complies | Complies |
| Colour of solution | Colourless, NMT B9 | Complies | Complies | Complies | Complies | Complies | Complies | Complies |
| pH | 6.5-8.0 | Complies | Complies | Complies | Complies | Complies | Complies | Complies |
| Assay cobitolimod | 90-110% | 101 | 103 | 104 | 105 | 108 | 106 | 108 |

| Purity of cobitolimod (HPLC)* | | | | | | | | |
|---|---|---|---|---|---|---|---|---|
| Sum of impurities | ≤10.0 area% | 5.5 | 5.2 | 5.1 | 5.4 | 5.3 | 5.3 | 5.6 |
| Purity | ≥ 90.0 area% | 94.5 | 94.8 | 94.9 | 94.7 | 94.7 | 94.7 | 94.4 |

| Main degradation impurity | | | | | | | | |
|---|---|---|---|---|---|---|---|---|
| - RRT 0.96-0.98 (N-3'PO/N-5'PO) | ≤4.0 area% | 2.2 | 2.3 | 1.8 | 2.0 | 2.0 | 2.0 | 2.4 |

| Purity of preservatives | | | | | | | | |
|---|---|---|---|---|---|---|---|---|
| p-hydroxybenzoic acid | Report (mg/mL) | 0.01 | 0.01 | 0.01 | 0.01 | 0.01 | 0.01 | 0.01 |

| Microbial quality (Ph. Eur.5.1.4) | | | | | | | | |
|---|---|---|---|---|---|---|---|---|
| TAMC | ≤10³ cfu/g | <10 | Nd | Nd | Nd | <1 | Nd | <1 |
| TYMC | ≤10² cfu/g | <10 | Nd | Nd | Nd | <1 | Nd | <1 |
| Evaporation, change of weight | ≤5.0% | - | 0.2 | 0.2 | 0.1 | 0.2 | 0.2 | 0.0 |

| | | | | | | | | |
|---|---|---|---|---|---|---|---|---|
| Nd: Not determined. * Reporting threshold: ≥0.15 area% | | | | | | | | |

**Table 11. Stability cobitolimod rectal solution 500 mg/50 mL, batch V3045 (25°C/40%RH)**

| **Test** | **Acceptance criteria** | **Initial** | **1 m** | **3 m** | **6 m** | **9 m** | **12 m** |
|---|---|---|---|---|---|---|---|
| Appearance | Clear solution | Complies | Complies | Complies | Complies | Complies | Complies |
| Colour of solution | Colourless, NMT B9 | Complies | Complies | Complies | Complies | Complies | Complies |
| pH | 6.5-8.0 | Complies | Complies | Complies | Complies | Complies | Complies |
| Assay cobitolimod | 90-110% | 101 | 103 | 101 | 102 | 104 | 104 |

| Purity of cobitolimod (HPLC)* | | | | | | | |
|---|---|---|---|---|---|---|---|
| ) Sum of impurities | ≤10.0 area% | 5.5 | 5.8 | 5.3 | 6.0 | 6.6 | 7.0 |
| Purity | ≥ 90.0 area% | 94.5 | 94.2 | 94.8 | 94.0 | 93.4 | 93.0 |

| **Test** | **Acceptance criteria** | **Initial** | **1 m** | **3 m** | **6 m** | **9 m** | **12 m** |
|---|---|---|---|---|---|---|---|
| Main degradation impurity | | | | | | | |
| - RRT 0.96-0.98 (N-3'PO/N-5'PO) | ≤4.0 area% | 2.2 | 2.5 | 2.6 | 2.6 | 3.0 | 3.3 |

| Purity of preservatives | | | | | | | |
|---|---|---|---|---|---|---|---|
| p-hydroxybenzoic acid | Report (mg/mL) | 0.01 | 0.01 | 0.02 | 0.04 | 0.05 | 0.06 |

| Microbial quality (Ph. Eur.5.1.4) | | | | | | | |
|---|---|---|---|---|---|---|---|
| TAMC | ≤10³ cfu/g | <10 | Nd | Nd | Nd | Nd | <1 |
| TYMC | ≤10² cfu/g | <10 | Nd | Nd | Nd | Nd | <1 |
| Evaporation, change of weight | ≤5.0% | - | -0.1 | -0.4 | -0.7 | -0.7 | -1.4 |

| | | | | | | | |
|---|---|---|---|---|---|---|---|
| Nd: Not determined. * Reporting threshold: ≥0.15 area% | | | | | | | |

**Table 12. Stability cobitolimod rectal solution 500 mg/50 mL, batch V3045 (40°C/25%RH)**

| **Test** | **Acceptance criteria** | **Initial** | **1 m** | **3 m** | **6 m** |
|---|---|---|---|---|---|
| Appearance | Clear solution | Complies | Complies | Complies | Complies |
| Colour of solution | Colourless, NMT B9 | Complies | Complies | Complies | Complies |
| pH | 6.5-8.0 | Complies | Complies | Complies | Complies |
| Assay cobitolimod | 90-110% (225.0 - 275.0 mg/50mL) | 101 (506.8) | 102 (511.2) | 100 (497.7) | 97 (486.2) |

| Purity of cobitolimod (HPLC)* | | | | | |
|---|---|---|---|---|---|
| Sum of impurities | ≤10.0 area% | 5.5 | 6.0 | 6.4 | 8.9 |
| Purity of cobitolimod | ≥ 90.0 area% | 94.5 | 94.0 | 93.7 | 91.2 |

| Main degradation impurity | | | | | |
|---|---|---|---|---|---|
| - RRT 0.96-0.98 (N-3'PO/N-5'PO) | 4.0 area% | 2.2 | 2.6 | 2.8 | 2.8 |
| Purity of preservatives p-hydroxybenzoic acid | Report (mg/mL) | 0.01 | 0.05 | 0.12 | 0.20 |

| Microbial quality (Ph. Eur.5.1.4.) | | | | | |
|---|---|---|---|---|---|
| TAMC | ≤10³ cfu/g | <10 | Nd | Nd | Nd |
| TYMC | ≤10² cfu/g | <10 | Nd | Nd | Nd |
| Evaporation, change of weight | ≤5.0% | - | -0.6 | -1.8 | -3.4 |

| | | | | | |
|---|---|---|---|---|---|
| Nd: Not determined. * Reporting threshold: ≥0.15 area% | | | | | |

**Table 13. Stability cobitolimod rectal solution, 250 mg/50 mL, batch V3080 (5°C/ambient RH)**

| **Test** | **Acceptance criteria** | **Initial** | **3 m** | **6 m** | **9 m** | **12 m** | **18 m** |
|---|---|---|---|---|---|---|---|
| Appearance | Clear solution | Complies | Complies | Complies | Complies | Complies | Complies |
| Colour of solution | Colourless, NMT B9 | Complies | Complies | Complies | Complies | Complies | Complies |
| pH | 6.5-8.0 | Complies | Complies | Complies | Complies | Complies | Complies |
| Assay cobitolimod | 90-110% | 103 | 106 | 106 | 106 | 106 | 107 |

| Purity of cobitolimod (HPLC)* | | | | | | | |
|---|---|---|---|---|---|---|---|
| Sum of impurities | ≤10.0 area% | 4.3 | 4.5 | 4.4 | 4.6 | 4.2 | 4.5 |
| Purity of cobitolimod | ≥ 90.0 area% | 95.8 | 95.5 | 95.6 | 95.4 | 95.8 | 95.5 |

| Main degradation impurity | | | | | | | |
|---|---|---|---|---|---|---|---|
| - RRT 0.96-0.98 (N-3'PO/N-5'PO) | ≤4.0 area% | 1.1 | 1.3 | 1.3 | 1.5 | 1.1 | 1.4 |

| Purity of preservatives | | | | | | | |
|---|---|---|---|---|---|---|---|
| p-hydroxybenzoic acid | Report (mg/mL) | 0.01 | 0.01 | 0.01 | 0.01 | 0.01 | 0.01 |

| Microbial quality (Ph. Eur.5.1.4.) | | | | | | | |
|---|---|---|---|---|---|---|---|
| TAMC | ≤10³ cfu/g | <1 | Nd | Nd | Nd | <1 | Nd |
| TYMC | ≤10² cfu/g | <1 | Nd | Nd | Nd | <1 | Nd |
| Evaporation, change of weight | ≤5.0% | - | 0.1 | 0.2 | 0.2 | 0.2 | 0.1 |

| | | | | | | | |
|---|---|---|---|---|---|---|---|
| Nd: Not determined. * Reporting threshold: ≥0.15 area% | | | | | | | |

**Table 14. Stability cobitolimod rectal solution, 250 mg/50 mL, batch V3080 (25°C/40%RH)**

| **Test** | **Acceptance criteria** | **Initial** | **1 m** | **3 m** | **6 m** |
|---|---|---|---|---|---|
| Appearance | Clear solution | Complies | Complies | Complies | Complies |
| Colour of solution | Colourless, NMT B9 | Complies | Complies | Complies | Complies |
| pH | 6.5-8.0 | Complies | Complies | Complies | Complies |
| Assay cobitolimod | 90-110% | 103 | 104 | 104 | 104 |

| Purity of cobitolimod (HPLC)* | | | | | |
|---|---|---|---|---|---|
| Sum of impurities | ≤10.0 area% | 4.3 | 4.2 | 5.1 | 5.4 |
| Purity of cobitolimod | ≥ 90.0 area% | 95.8 | 95.8 | 94.9 | 94.6 |

| Main degradation impurity | | | | | |
|---|---|---|---|---|---|
| - RRT 0.96-0.98 (N-3'PO/N-5'PO) | ≤4.0 area% | 1.1 | 1.3 | 1.9 | 2.3 |
| Purity of preservatives p-hydroxybenzoic acid | Report (mg/mL) | 0.01 | 0.01 | 0.02 | 0.04 |

| Microbial quality (Ph. Eur.5.1.4.) | | | | | |
|---|---|---|---|---|---|
| TAMC | ≤10³ cfu/g | <1 | Nd | Nd | <1 |
| TYMC | ≤10² cfu/g | <1 | Nd | Nd | <1 |
| Evaporation, change of weight | ≤5.0% | - | -0.1 | -0.3 | -0.8 |

| | | | | | |
|---|---|---|---|---|---|
| Nd: Not determined. * Reporting threshold: ≥0.15 area% | | | | | |

**Table 15. Stability cobitolimod rectal solution, 250 mg/50 mL, batch V3080 (40°C/25%RH)**

| **Test** | **Acceptance criteria** | **Initial** | **1 m** | **3 m** |
|---|---|---|---|---|
| Appearance | Clear solution | Complies | Complies | Complies |
| Colour of solution | Colourless, NMT B9 | Complies | Complies | Complies |
| pH | 6.5-8.0 | Complies | Complies | Complies |
| Assay cobitolimod | 90-110% | 103 | 102 | 102 |

| Purity of cobitolimod (HPLC)* | | | | |
|---|---|---|---|---|
| Sum of impurities | ≤10.0 area% | 4.3 | 5.0 | 6.2 |
| Purity of cobitolimod | ≥ 90.0 area% | 95.8 | 95.0 | 93.9 |
| Main degradation impurity | | | | |
| - RRT 0.96-0.98 (N-3'PO/N-5'PO) | 4.0 area% | 1.1 | 1.8 | 2.4 |

| Purity of preservatives | | | | |
|---|---|---|---|---|
| p-hydroxybenzoic acid | Report (mg/mL) | 0.01 | 0.05 | 0.13 |

| Microbial quality (Ph. Eur.5.1.4.) | | | | |
|---|---|---|---|---|
| TAMC | ≤10³ cfu/g | <1 | Nd | <1 |
| TYMC | ≤10² cfu/g | <1 | Nd | <1 |
| Evaporation, change of weight | ≤5.0% | - | -0.7 | -2.0 |

| | | | | |
|---|---|---|---|---|
| Nd: Not determined. * Reporting threshold: ≥0.15 area% | | | | |

**Table 16. Stability cobitolimod rectal solution, 500 mg/50 mL, batch V3081 (5°C/ambient RH)**

| **Test** | **Acceptance criteria** | **Initial** | **3 m** | **6 m** | **9 m** | **12 m** | **18 m** |
|---|---|---|---|---|---|---|---|
| Appearance | Clear solution | Complies | Complies | Complies | Complies | Complies | Complies |
| Colour of solution | Colourless, NMT B9 | Complies | Complies | Complies | Complies | Complies | Complies |
| pH | 6.5-8.0 | Complies | Complies | Complies | Complies | Complies | Complies |
| Assay cobitolimod | 90-110% | 103 | 105 | 106 | 107 | 106 | 108 |

| Purity of cobitolimod (HPLC)* | | | | | | | |
|---|---|---|---|---|---|---|---|
| Sum of impurities | ≤10.0 area% | 4.3 | 4.4 | 4.4 | 4.6 | 4.3 | 4.5 |
| Purity of cobitolimod | ≥ 90.0 area% | 95.7 | 95.6 | 95.7 | 95.4 | 95.7 | 95.5 |

| Main degradation impurity | | | | | | | |
|---|---|---|---|---|---|---|---|
| - RRT 0.96-0.98 (N-3'PO/N-5'PO) | ≤4.0 area% | 1.1 | 1.3 | 1.3 | 1.4 | 1.1 | 1.4 |

| Purity of preservatives | | | | | | | |
|---|---|---|---|---|---|---|---|
| p-hydroxybenzoic acid | Report (mg/mL) | 0.01 | 0.01 | 0.03 | 0.01 | 0.01 | 0.01 |

| Microbial quality (Ph. Eur.5.1.4.) | | | | | | | |
|---|---|---|---|---|---|---|---|
| TAMC | ≤10³ cfu/g | <1 | Nd | Nd | Nd | <1 | Nd |

| **Test** | **Acceptance criteria** | **Initial** | **3 m** | **6 m** | **9 m** | **12 m** | **18 m** |
|---|---|---|---|---|---|---|---|
| TYMC | ≤10² cfu/g | <1 | Nd | Nd | Nd | <1 | Nd |
| Evaporation, change of weight | ≤5.0% | - | 0.1 | 0.2 | 0.2 | 0.2 | 0.1 |

| | | | | | | | |
|---|---|---|---|---|---|---|---|
| Nd: Not determined. * Reporting threshold: ≥0.15 area% | | | | | | | |

**Table 17. Stability cobitolimod rectal solution, 500 mg/50 mL, batch V3081 (25°C/40%RH)**

| **Test** | **Acceptance criteria** | **Initial** | **1 m** | **3 m** | **6 m** |
|---|---|---|---|---|---|
| Appearance | Clear solution | Complies | Complies | Complies | Complies |
| Colour of solution | Colourless, NMT B9 | Complies | Complies | Complies | Complies |
| pH | 6.5-8.0 | Complies | Complies | Complies | Complies |
| Assay cobitolimod | 90-110% | 103 | 104 | 105 | 106 |

| Purity of cobitolimod (HPLC)* | | | | | |
|---|---|---|---|---|---|
| Sum of impurities | ≤10.0 area% | 4.3 | 4.4 | 4.8 | 5.2 |
| Purity of cobitolimod | ≥ 90.0 area% | 95.7 | 95.6 | 95.3 | 94.8 |

| Main degradation impurity | | | | | |
|---|---|---|---|---|---|
| - RRT 0.96-0.98 (N-3'PO/N-5'PO) | ≤4.0 area% | 1.1 | 1.3 | 1.7 | 2.1 |

| Purity of preservatives | | | | | |
|---|---|---|---|---|---|
| p-hydroxybenzoic acid | Report (mg/mL) | 0.01 | 0.01 | 0.02 | 0.06 |

| Microbial quality (Ph. Eur.5.1.4.) | | | | | |
|---|---|---|---|---|---|
| TAMC | ≤10³ cfu/g | <1 | Nd | Nd | <1 |
| TYMC | ≤10² cfu/g | <1 | Nd | Nd | <1 |
| Evaporation, change of weight | ≤5.0% | - | -0.1 | -0.2 | -0.8 |

| | | | | | |
|---|---|---|---|---|---|
| Nd: Not determined. * Reporting threshold: ≥0.15 area% | | | | | |

**Table 18. Stability cobitolimod rectal solution, 500 mg/50 mL, batch V3081 (40°C/25%RH)**

| **Test** | **Acceptance criteria** | **Initial** | **1 m** | **3 m** |
|---|---|---|---|---|
| Appearance | Clear solution | Complies | Complies | Complies |
| Colour of solution | Colourless, NMT B9 | Complies | Complies | Complies |
| pH | 6.5-8.0 | Complies | Complies | Complies |
| Assay cobitolimod | 90-110% | 103 | 102 | 103 |
| Purity of cobitolimod (HPLC)* | | | | |
| Sum of impurities | ≤10.0 area% | 4.3 | 4.5 | 5.7 |
| Purity of cobitolimod | ≥ 90.0 area% | 95.7 | 95.5 | 94.3 |
| Main degradation impurity | | | | |
| - RRT 0.96-0.98 (N-3'PO/N-5'PO) | ≤4.0 area% | 1.1 | 1.5 | 1.9 |
| Purity of preservatives | | | | |
| p-hydroxybenzoic acid | Report (mg/mL) | 0.01 | 0.04 | 0.12 |
| Microbial quality (Ph. Eur.5.1.4.) | | | | |
| TAMC | ≤10³ cfu/g | <1 | Nd | <1 |
| TYMC | ≤10² cfu/g | <1 | Nd | <1 |
| Evaporation, change of weight | ≤5.0% | - | -0.7 | -2.0 |

| | | | | |
|---|---|---|---|---|
| Nd: Not determined. * Reporting threshold: ≥0.15 area% | | | | |

### Conclusion

Stability data for cobitolimod rectal solution at 250 mg/50 mL and 500 mg/50 mL were obtained. The results show that a cobitolimod rectal solution according to the present invention, at concentrations of both 250 mg/50 mL and 500 mg/50mL, is stable in both ambient conditions and when exposed to high temperature and/or humidity. Both concentrations show similar stability across the range of tested conditions.

### Example 4 - Comparative stability study in pre-filled enemas

The stability of cobitolimod in water (as described in, for example, WO 2021/037764) and in the present enema formulation was compared. In addition, stability was also evaluated in preserved water (water + paraben). The composition of the respective formulations is shown in Table 19.

Each formulation was filled into enema bottles and placed on stability study for up to 12 months at 40°C +/- 2°C (75 % RH +/- 5% RH).

**Table 19. Composition of formulations used in the comparative stability study in enema bottles**

| | Batch V3131 (water) | Batch V3132 (preserved water) | Batch V3133 (present enema formulation) |
|---|---|---|---|
| Cobitolimod | X | X | X |
| Sodium methylparaben | - | X | X |
| Sodium propylparaben | - | X | X |
| Citric acid anhydrous | - | - | X |
| Disodium hydrogenphosphate dihydrate | - | - | X |
| Water | x | X | x |

Results are presented in Table 20.

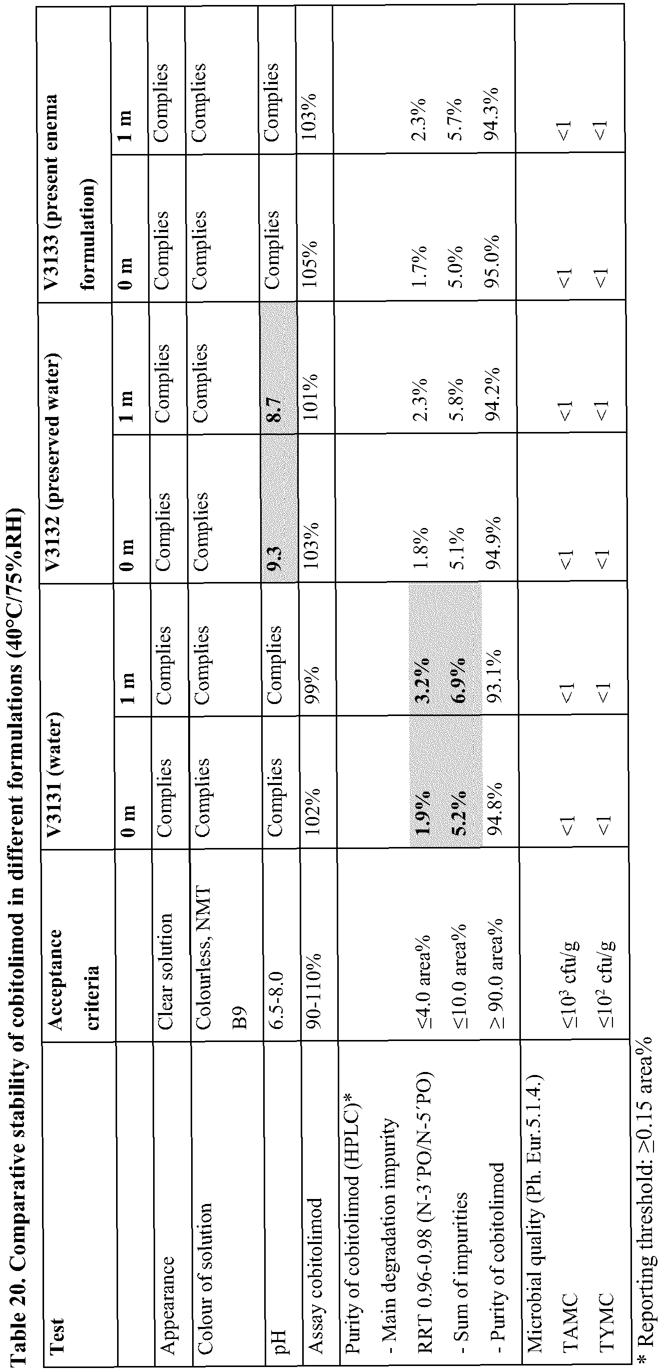

### Conclusion

Data from the main degradation impurity (RRT 0.96-0.98 (N-3'PO/N-5'PO)) and from total impurities shows that cobitolimod is more stable in the formulation of the present invention than it is in a formulation of cobitolimod and water.

In addition, the data shows that degradation does not arise solely from any potential microbial contamination in the bottle, but also from oxidation. In particular, the pH data shows that the formulation with preserved water (which eliminates any issues with possible microbial contamination) still degraded to an unacceptable level. In contrast, the formulation of the present invention did not degrade in the same way.

## Claims

1. An aqueous formulation suitable for rectal administration comprising (i) an oligonucleotide comprising the sequence 5'-GGAACAGTTCGTCCATGGC-3' (SEQ ID NO:2), (ii) at least one preservative and (iii) a buffer comprising citric acid and a phosphate salt.

2. The formulation according to claim 1 wherein the at least one preservative comprises one or more parabens,
preferably wherein the at least one preservative is selected from the group consisting of Na-methylparaben, Na-propylparaben and mixtures thereof.

3. The formulation according to any preceding claim wherein the formulation has a pH in the range of 6.0 to 8.0,
preferably wherein the formulation has a pH in the range of 6.5 to 8.0.

4. The formulation according to any preceding claim wherein the phosphate salt is disodium hydrogenphosphate.

5. The formulation according to any preceding claim, wherein said oligonucleotide has the sequence 5'-GGAACAGTTCGTCCATGGC-3' (SEQ ID NO:2), wherein the CG dinucleotide is unmethylated,
preferably wherein said oligonucleotide has the sequence 5'-G*G*A*ACAGTTCGTCCAT*G*G*C-3' (SEQ ID NO:1), wherein the CG dinucleotide is unmethylated,
more preferably wherein the oligonucleotide is cobitolimod.

6. The formulation according to any preceding claim wherein the oligonucleotide is present in the formulation at a concentration of from 0.5 to 15 mg/mL,
preferably wherein the oligonucleotide is present in the formulation at a concentration of from 2.5 to 12.5 mg/mL.

7. A pre-filled enema suitable for self-administration, said pre-filled enema comprising:
(a) a container; and within the container
(b) the formulation as defined in any one of claims 1 to 6.

8. The pre-filled enema according to claim 7 wherein the container comprises a squeezable bottle,
preferably wherein the squeezable bottle is a concertina-shaped bottle.

9. The pre-filled enema according to claim 8 wherein the bottle is formed from polyethylene, preferably wherein the bottle is formed from low-density polyethylene.

10. The pre-filled enema according to any one of claims 7 to 9 wherein the container further comprises an applicator suitable for dispensing the formulation,
preferably wherein the applicator comprises a tip suitable for insertion into the rectum.

11. The pre-filled enema according to claim 10 wherein the applicator is formed from polyethylene,
preferably wherein the applicator is formed from low density polyethylene.

12. The pre-filled enema according to any one of claims 7 to 11 wherein the amount of formulation within the container is from 20 to 60 mL,
preferably wherein the amount of formulation within the container is about 50 mL.

13. A pack comprising one or more pre-filled enemas as defined in any one of claims 7 to 12.

14. A formulation according to any one of claims 1 to 6 for use in the treatment of an inflammatory bowel disease,
preferably wherein said inflammatory bowel disease is ulcerative colitis or Crohn's disease.

15. The formulation for use according to claim 14 wherein the formulation is administered rectally using the pre-filled enema as defined in any one of claims 7 to 12,
preferably wherein the formulation is self-administered using the pre-filled enema as defined in any one of claims 7 to 12.

## Patentansprüche

1. Wässrige Formulierung, die für die rektale Verabreichung geeignet ist und (i) ein Oligonukleotid, umfassend die Sequenz 5'-GGAACAGTTCGTCCATGGC-3' (SEQ ID NO:2), (ii) wenigstens ein Konservierungsmittel und (iii) einen Puffer umfasst, der Zitronensäure und ein Phosphatsalz umfasst.

2. Formulierung nach Anspruch 1, wobei das wenigstens eine Konservierungsmittel ein oder mehrere Parabene umfasst,
vorzugsweise wobei das wenigstens eine Konservierungsmittel aus der Gruppe ausgewählt ist, die aus Na-Methylparaben, Na-Propylparaben und Mischungen davon besteht.

3. Formulierung nach einem der vorhergehenden Ansprüche, wobei die Formulierung einen pH-Wert in dem Bereich von 6,0 bis 8,0 aufweist,
vorzugsweise wobei die Formulierung einen pH-Wert in dem Bereich von 6,5 bis 8,0 aufweist.

4. Formulierung nach einem der vorhergehenden Ansprüche, wobei das Phosphatsalz Dinatriumhydrogenphosphat ist.

5. Formulierung nach einem der vorhergehenden Ansprüche, wobei das Oligonukleotid die Sequenz 5'-GGAACAGTTCGTCCATGGC-3' (SEQ ID NO:2) aufweist, wobei das CG-Dinukleotid unmethyliert ist,
vorzugsweise wobei das Oligonukleotid die Sequenz 5'-G*G*A*ACAGTTCGTCCAT*G*G*C-3' (SEQ ID NO:1) aufweist, wobei das CG-Dinukleotid nicht methyliert ist,
stärker bevorzugt wobei das Oligonukleotid Cobitolimod ist.

6. Formulierung nach einem der vorhergehenden Ansprüche, wobei das Oligonukleotid in der Formulierung in einer Konzentration von 0,5 bis 15 mg/ml vorliegt,
vorzugsweise wobei das Oligonukleotid in der Formulierung in einer Konzentration von 2,5 bis 12,5 mg/ml vorhanden ist.

7. Vorgefüllter Einlauf, der für die Selbstverabreichung geeignet ist, wobei der vorgefüllte Einlauf umfasst:
(a) einen Behälter; und innerhalb des Behälters
(b) die Formulierung nach einem der Ansprüche 1 bis 6.

8. Vorgefüllter Einlauf nach Anspruch 7, wobei der Behälter eine zusammendrückbare Flasche umfasst,
vorzugsweise wobei die zusammendrückbare Flasche eine ziehharmonikaförmige Flasche ist.

9. Vorgefüllter Einlauf nach Anspruch 8, wobei die Flasche aus Polyethylen ausgebildet ist,
vorzugsweise wobei die Flasche aus Polyethylen niedriger Dichte ausgebildet ist.

10. Vorgefüllter Einlauf nach einem der Ansprüche 7 bis 9, wobei der Behälter ferner einen Applikator umfasst, der für die Abgabe der Formulierung geeignet ist,
vorzugsweise wobei der Applikator eine zum Einführen in das Rektum geeignete Spitze umfasst.

11. Vorgefüllter Einlauf nach Anspruch 10, wobei der Applikator aus Polyethylen ausgebildet ist,
vorzugsweise wobei der Applikator aus Polyethylen niedriger Dichte ausgebildet ist.

12. Vorgefüllter Einlauf nach einem der Ansprüche 7 bis 11, wobei die Menge der Formulierung in dem Behälter von 20 bis 60 ml beträgt,
vorzugsweise wobei die Menge der Formulierung innerhalb des Behälters etwa 50 ml beträgt.

13. Packung, umfassend einen oder mehrere vorgefüllte Einläufe nach einem der Ansprüche 7 bis 12.

14. Formulierung nach einem der Ansprüche 1 bis 6 für die Verwendung bei der Behandlung einer entzündlichen Darmerkrankung,
vorzugweise wobei die entzündliche Darmerkrankung Colitis ulcerosa oder Morbus Crohn ist.

15. Formulierung für die Verwendung nach Anspruch 14, wobei die Formulierung rektal unter Verwendung des vorgefüllten Einlaufs nach einem der Ansprüche 7 bis 12 verabreicht wird,
vorzugsweise wobei die Formulierung unter Verwendung des vorgefüllten Einlaufs nach einem der Ansprüche 7 bis 12 selbst verabreicht wird.

## Revendications

1. Formulation aqueuse adaptée à l'administration rectale comprenant (i) un oligonucléotide comprenant la séquence 5'-GGAACAGTTCGTCCATGGC-3' (SEQ ID NO : 2), (ii) au moins un conservateur et (iii) un tampon comprenant de l'acide citrique et un sel de phosphate.

2. Formulation selon la revendication 1, dans laquelle l'au moins un conservateur comprend un ou plusieurs parabènes,
préférablement dans laquelle l'au moins un conservateur est choisi dans le groupe constitué par le Na-méthylparabène, le Na-propylparabène et leurs mélanges.

3. Formulation selon l'une quelconque des revendications précédentes, dans laquelle la formulation a un pH dans la plage de 6,0 à 8,0,
préférablement dans laquelle la formulation a un pH dans la plage de 6,5 à 8,0.

4. Formulation selon l'une quelconque des revendications précédentes, dans laquelle le sel de phosphate est l'hydrogénophosphate disodique.

5. Formulation selon l'une quelconque des revendications précédentes, dans laquelle ledit oligonucléotide a la séquence 5'-GGAACAGTTCGTCCATGGC-3' (SEQ ID NO : 2), dans laquelle le dinucléotide CG est non méthylé,
préférablement dans laquelle ledit oligonucléotide a la séquence 5'-G* G*A*ACAGTTCGTCCAT*G*G*C-3' (SEQ ID NO : 1), dans laquelle le dinucléotide CG est non méthylé,
plus préférablement dans laquelle l'oligonucléotide est le cobitolimod.

6. Formulation selon l'une quelconque des revendications précédentes, dans laquelle l'oligonucléotide est présent dans la formulation à une concentration de 0,5 à 15 mg/ml,
préférablement dans laquelle l'oligonucléotide est présent dans la formulation à une concentration de 2,5 à 12,5 mg/ml.

7. Lavement pré-rempli convenant à l'auto-administration, ledit lavement pré-rempli comprenant :
(a) un récipient ; et à l'intérieur du récipient
(b) la formulation telle que définie dans l'une quelconque des revendications 1 à 6.

8. Lavement pré-rempli selon la revendication 7, dans lequel le récipient comprend une bouteille compressible,
préférablement dans lequel la bouteille compressible est une bouteille en forme d'accordéon.

9. Lavement pré-rempli selon la revendication 8, dans lequel la bouteille est formée de polyéthylène, préférablement dans lequel la bouteille est formée de polyéthylène basse densité.

10. Lavement pré-rempli selon l'une quelconque des revendications 7 à 9, dans lequel le récipient comprend en outre un applicateur adapté pour distribuer la formulation,
préférablement dans lequel l'applicateur comprend une pointe adaptée pour l'insertion dans le rectum.

11. Lavement pré-rempli selon la revendication 10, dans lequel l'applicateur est formé de polyéthylène,
préférablement dans lequel l'applicateur est formé de polyéthylène basse densité.

12. Lavement pré-rempli selon l'une quelconque des revendications 7 à 11, dans lequel la quantité de formulation dans le récipient est de 20 à 60 ml,
préférablement dans lequel la quantité de formulation dans le récipient est d'environ 50 ml.

13. Pack comprenant un ou plusieurs lavements pré-remplis tels que définis dans l'une quelconque des revendications 7 à 12.

14. Formulation selon l'une quelconque des revendications 1 à 6 pour une utilisation dans le traitement d'une maladie intestinale inflammatoire,
préférablement dans laquelle ladite maladie intestinale inflammatoire est la colite ulcéreuse ou la maladie de Crohn.

15. Formulation pour une utilisation selon la revendication 14, dans laquelle la formulation est administrée par voie rectale en utilisant le lavement pré-rempli tel que défini dans l'une quelconque des revendications 7 à 12,
préférablement dans laquelle la formulation est auto-administrée en utilisant le lavement pré-rempli tel que défini dans l'une quelconque des revendications 7 à 12.
